# EUROPEAN PATENT SPECIFICATION

(11) **EP 2 594 134 B1**
(45) Date of publication and mention of the grant of the patent: **02.08.2017**
(21) Application number: 11807066.3
(22) Date of filing: 14.07.2011
(51) Int. Cl.: A01N 43/60, A01N 35/08, A01P 21/00, A01P 3/00

(54) **USE OF AN AGRICULTURAL AGENT CONTAINING 2,5-DIKETOPIPERAZINE DERIVATIVE AS ACTIVE INGREDIENT**
VERWENDUNG EINER AGROCHEMIKALIE MIT EINEM 2,5-DIKETOPIPERAZINDERIVAT ALS WIRKSTOFF
UTILISATION D'AGENT AGRICOLE CONTENANT UN DÉRIVÉ DE 2,5-DICÉTOPIPÉRAZINE EN TANT QU'INGRÉDIENT ACTIF

(30) Priority: 15.07.2010 KR 20100068418
(43) Date of publication of application: 22.05.2013
(73) Proprietor: Republic of Korea (Management: Rural Development Administration), Suwon, Gyeonggi-do 441-707 (KR); Napro Biotec Inc., 56 Gongjudaehak-ro, Gongju-si Chungcheongnam-do (KR)
(72) Inventor: PARK, Kyung Seok, Suwon-si Gyeonggi-do 442-190 (KR); PARK, Jin Woo, Suwon-si Gyeonggi-do 440-712 (KR); LEE, Sang Yeob, Suwon-si Gyeonggi-do 441-761 (KR); MOON, Surk Sik, Gongju-si Chungcheongnam-do 314-802 (KR); HONG, In Seok, Daejeon 305-509 (KR)
(74) Representative: WSL Patentanwälte Partnerschaft mbB
(86) International application number: PCT/KR2011/005198
(87) International publication number: WO 2012/008781

(56) References cited:
- WO-A1-2008/152074
- WO-A1-2010/049369
- WO-A2-2008/152072
- JP-A- S62 228 002
- KR-A- 20070 043 040
- KR-A- 20080 092 407
- US-A- 4 140 791
- US-A- 4 140 791
- DATABASE WPI Week 200781 Thomson Scientific, London, GB; AN 2007-873505 XP002717900, "Compound 3-Isopropyl-6-(-2-methyl butyl)piperazine-2,5-diketone and the preparation and application of it", & CN 1 974 560 A 6 June 2007 (2007-06-06)
- G. DEGRASSI ET AL: "Plant Growth-Promoting Pseudomonas putida WCS358 Produces and Secretes Four cyclic Dipeptides: Cross-Talk with Quorum Sensing Bacterial Sensors", CURRENT MICROBIOLOGY, vol. 45, no. 4, October 2002 (2002-10), pages 250-254, XP002717905, DOI: 10.1007/s00284-002-3704-y
- DATABASE CAPLUS [Online] CHEMICAL ABSTRACTS SERVICE, COLUMBUS, OHIO, US; 1996, KIMURA, YASUO ET AL: "Cyclo-(L-tryptophyl-L-phenylalanyl), a plant growth regulator produced by the fungus Penicillium.", XP002717901, retrieved from STN Database accession no. 1996:101419 & KIMURA, YASUO ET AL: "Cyclo-(L-tryptophyl-L-phenylalanyl), a plant growth regulator produced by the fungus Penicillium.", PHYTOCHEMISTRY , 41(3), 665-9 CODEN: PYTCAS; ISSN: 0031-9422, 1996, DOI: 10.1016/0031-9422(95)00693-1
- DATABASE CAPLUS [Online] CHEMICAL ABSTRACTS SERVICE, COLUMBUS, OHIO, US; Shenyang pharamaceutical University: "New cyclodipeptide used as drought resistant agent and plant growth regulator and its preparation method", XP002717902, Database accession no. 2008:86396
- DATABASE CAPLUS [Online] CHEMICAL ABSTRACTS SERVICE, COLUMBUS, OHIO, US; 1998, CRONAN, JOHN M., JR. ET AL: "Plant growth promoters isolated from a marine bacterium associated with Palythoa sp.", XP002717903, retrieved from STN Database accession no. 1998:307378 & CRONAN, JOHN M., JR. ET AL: "Plant growth promoters isolated from a marine bacterium associated with Palythoa sp.", NATURAL PRODUCT LETTERS , 11(4), 271-278 CODEN: NPLEEF; ISSN: 1057-5634, 1998, DOI: 10.1080/10575639808044959

## Description

### BACKGROUND

### (a) Technical Field

The present invention relates to the use of an agricultural agent containing a 2,5-diketopiperazine derivative for providing induced systemic resistance (ISR) against plant diseases.

### (b) Background Art

2,5-Diketopiperazine compounds have various biological activities [Prasad, C., Peptides 1995, 16, 151-164]. Particularly, the 2,5-diketopiperazine derivatives having the amino acid proline in the backbone are known to recognize various receptors and regulate their activities and to act as inhibitors of various enzymes [Wang, H. J., Med. Chem. 2000, 43, 577; Houston, D. R. J., Med. Chem. 2004, 47, 5713]. However, the 2,5-diketopiperazine derivatives' ability of inhibiting the proliferation of pathogens by inducing expression of pathogen-resistant genes in plants has never been reported in literatures and was first discovered by the inventors of the present invention.

When invaded by pathogens or physically damaged, plants produce signal transducing molecules to protect themselves [Stout M. J.; Fidantsef, A. L.; Duffey, S. S.; Bostock, R. M., Physiology and Molecular Plant Pathology, 1999, 54, 115-130]. Salicylic acid and jasmonic acid are representative signal transducing molecules of plants. When a plant defense mechanism is turned on against external invasion, the signal transducing molecule salicylic acid triggers expression of the genes such as PR-1, BGL-2, PR-5, SID-2, EDS-5 and PAD-4 in the plant. Meanwhile, jasmonic acid induces the expression of genes such as PDF1.2, VSP, HEL, THI-2, FAD3, ERS1 and ERF1 [Dong, X., Current Opinion in Plant Biology, 1998, 1, 316-323; Glazebrook, J., Current Opinion in Planet Biology, 1999, 2, 280-286; Bostock, R. M., Physiology and Molecular Plant Pathology, 1999, 55, 99-109]. In particular, the PR-1 gene is a marker gene related with resistance to pathogens induced by salicylic acid. The expression of the PR-1 gene is a clear evidence of signal transduction in plants. When the PR protein is produced, a plant acquires resistance to pathogens including bacteria. Additionally, the PDF1.2 gene is a marker gene related with resistance to pathogens in plants induced by jasmonic acid. The expression of the PDF1.2 gene is a clear evidence of signal transduction for dense mechanism in plants [Reymond, P.; Farmer E. E., Currenf Opinion in Plant Biology, 1998, 1, 404-411].

Commercially available plant signal transducing molecules include benzo-1,2,3-thiadiazole-7-carbothioic acid S-methyl ester (BTH). BTH is a synthetic compound similar to salicylic acid in chemical structure and is reported to induce resistance to *Blumeria graminis* causing powdery mildew in barley [Faroro, F.; Maffi, D.; Cantu, D.; Iriti, M., Biocontrol, 2008, 53, 387-401] and ozone resistance in plants [Iriti, M.; Rabotti, G.; Ascensao, A.; Faoro, F., J. Agric Food Chem. 2003, 51, 4308-4314), induce resistance to *Botrytis cinerea* causing gray mold and promote biosynthesis of resveratrol and anthocyanin in grapes [Iriti, M.; Rossoni, M.; Borgo, M.; Faoro, F., J. Agric Food Chem. 2004, 52, 4406-4413] and induce resistance to powdery mildew and accumulation of phenolics in strawberry [Hukkanen, A.; Kokko, H. I.; Buchala, A. J.; McDougall, G. J.; Stewart, D.; Karenlamp, S. O.; Karjalainen, R. O., J. Agric Food Chem. 2007, 55, 1862-1870]. BTH induces the expression of the PR-1 gene as salicylic acid but it does not induce the expression of thePDF1.2 gene.

As described above, when self-defending signal transducing molecules such as salicylic acid and jasmonic acid induces the expression of genes such as PR-1 or PDF1.2, the plant can inhibit the proliferation of pathogens and endure physical damage. That is to say, a self-dense ability is induced in the plant. Accordingly, since the plant acquires resistance to pathogens without help from antimicrobial or bactericidal agents, a substance capable of expressing the genes is of great value as an agricultural agent.

2,5-Diketopiperazine derivatives included in the agricultural agent of the present invention as active ingredient are natural substances found in roasted coffee bean (Ginz, M.; Engelhardt, U. H., J Agric. Food Chem., 2000, 48, 3528-3532), cacao fruit (Stark, T.; Hofmann, T., J Agric. Food Chem., 2005, 53, 7222-7231), beer (Gautschi, M.; Schmid, J. P.; Peppard, T. L.; Ryan, T. P.; Tuorto, R.; Yang, X., J Agric. Food Chem., 1997, 45, 3183-3189), cooked beef (Chen, M. Z.; Dewis, M. L.; Kraut, K.; Merrit, D.; Reiber, L., Trinnaman, L.; Da Costa, N. C., J. Food Sci., 2009, 74, C100-C105). Accordingly, it is likely that an agricultural agent containing a 2,5-diketopiperazine derivative as an active ingredient will be useful as an environment-friendly agricultural agent with very little toxicity.

The inventors of the present invention have discovered for the first time that the use of 2,5-diketopiperazine derivatives induces the plant pathogen-resistant genes PR-1 and PDF1.2 as signal transducing molecules in plants, exhibiting the effect of controlling plant diseases even when not treated directly on the lesion site and promoting growth of the plants.

### SUMMARY

The present invention is directed to providing a use of 2,5-diketopiperazine derivatives as agricultural agent for providing induced systemic resistance (ISR) against plant diseases according to claim **1**.

In an aspect, an agricultural agent containing a compound selected from a 2,5-diketopiperazine derivative represented by Chemical Formula **1** and an agriculturally acceptable salt thereof as an active ingredient is described: 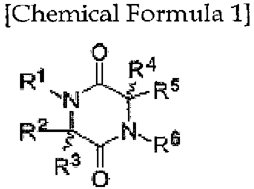 where
each of R¹ and R⁶, which arc identical or different, is a hydrogen atom or a C₁-C₆ linear or branched alkyl group;
each of R², R³, R⁴ and R⁵, which are identical or different, is a hydrogen atom or a C₁-C₆ linear or branched alkyl group substituted or unsubstituted with a substituent selected from hydroxy, mercapto, amino, guanidino, carbamoyl, carboxyl, C₁-C₆ alkoxycarbonyl, C₁-C₆ alkylthio, tritylthio, acetylamino, phenyl, hydroxyphenyl, imidazolyl and indolyl,
wherein one of R² and R³ may be linked with R¹ to form a 5- to 7-membered fused ring or one of R⁴ and R⁵ may be linked with R⁶ to form a 5- to 7-membered fused ring.

Since the 2,5-diketopiperazine derivative represented by Chemical Formula 1 has at least one chiral carbon, the agricultural agent may contain the compound represented by Chemical Formula 1 as a racemate or an isomer.

The agricultural agent promotes the growth of stem, leaves, root, etc. of plants.When treated to plants, the use of the agricultural agent of the present invention remarkably reduces lesions by inducing production of plant disease resistance proteins through expression of the pathogen-resistance genes PR-1 and PDF1.2 and thereby inhibiting infection and proliferation of pathogens. That is to say, the use of agricultural agent of the present invention induces resistance to pathogens in plants through self-defense mechanism.

Further, the use of the agricultural agent of the present invention exhibits the effect of controlling plant diseases even at untreated sites by continuously inducing the self-defense mechanism of plants. For example, if the agent is treated on the root, stem or leaf a plant, its effect is exerted also in the fruit.

Since the use of the agricultural agent of the present invention exhibits excellent effect of controlling plant diseases caused by bacteria, viruses or fungi such as soft rot, damping off, blight, withering, spot or mosaic disease, it is useful as an environment-friendly agricultural agent capable of replacing the existing microbicidal agents.

The use of the agricultural agent of the present invention exhibits superior efficacy in Solanaceae family plants such as potato, red pepper, tomato, Cucurbitaceae family plants such as cucumber, watermelon, melon, Chinese Brassicaceae family plants such as Chinese cabbage, lettuce, radish, cabbage, celery, perilla, strawberry, green onion, garlic, ginger, onion, and so forth.

Other features and aspects of the present invention will be apparent from the following detailed description, drawings and claims.

### BRIEF DESCRIPTION OF THE DRAWINGS

The above and other objects, features and advantages of the present invention will now be described in detail with reference to certain exemplary embodiments thereof illustrated in the accompanying drawings which are given hereinbelow by way of illustration only, and thus are not limitative of the invention, and wherein:
FIG. 1 shows the activity of the pathogen resistance inducing gene PR-1 in thale cress after treatment with Com. No. 26, 27, 24, 35,23,43,44 and 34;
FIG. 2 shows the expression of the PR-1 and PDF1.2 genes in thale cress after treatment with Compound No. 29;
FIG. 3 shows the expression of the PR-1 and PDF1.2 genes in thale cress after treatment with Compound No. 33;
FIG. 4 shows the effect of inhibition of soft rot in tobacco leaves caused by *Erwinia carotovora* SCC1 after treatment with Com. No. 1, 2, 3, 4 and 5;
FIG. 5 shows the effect of inhibition of soft rot in tobacco leaves caused by *Pectobacterium carotovorum* after treatment with Com. No. 25, 28, 29, 30 and 31;
FIG. 6 shows the effect of inhibition of soft rot in tobacco leaves caused by *Pectobacterium carotovorum* SCC1 after treatment with Com. No. 56, 57, 58, 60, 61, 62, 63 and 67;
FIG. 7 shows the effect of inhibition of soft rot in tobacco leaves caused by *Pectobacterium carotovorum* after treatment with Com. No. 24, 25, 26, 27, 29 and 68;
FIG. 8 shows the effect of inhibition of lesion in tobacco leaves and cucumber leaves caused by *Pectobacterium carotovorum* SCC1 after treatment with Compound No. 3;
FIG. 9 shows the effect of inhibition of lesion in red pepper fruits caused by *Colletotrichum acutatum* after treatment with Compound No. 30;
FIG. 10 shows the effect of inhibition of lesion in Chinese cabbage leaves due to bacterial soft rot caused by *Pectobacterium carotovorum* SCC1 after treatment with Com. No. 25, 26 and 29; and

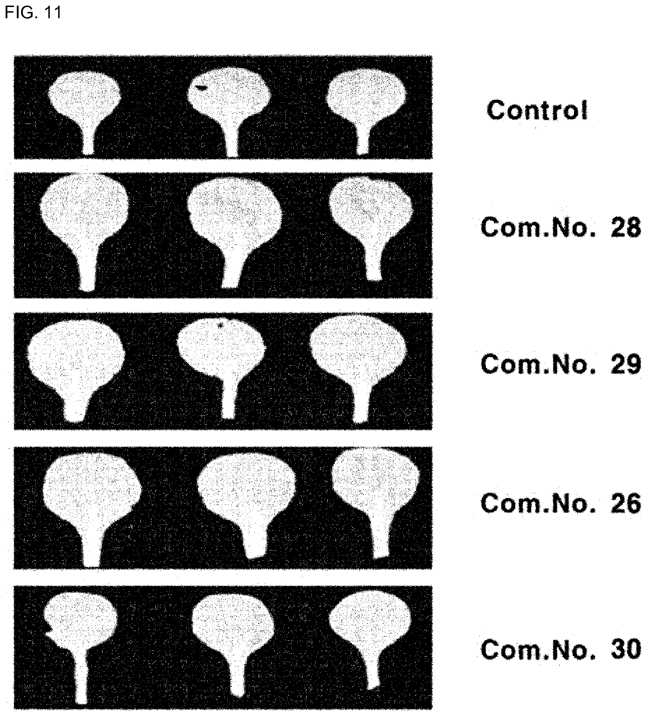

FIG. 11 shows the effect of promotion of Chinese cabbage leaf growth after treatment with Com. No. 28, 29, 26 and 30 at 100 ppm.

### DETAILED DESCRIPTION

Hereinafter, reference will now be made in detail to various embodiments of the present invention, examples of which are illustrated in the accompanying drawings and described below.

An active ingredient comprising a 2,5-diketopiperazine derivative represented by Chemical Formula 1 or an agriculturally acceptable salt thereof as an agricultural agent is described.

As used in the present invention, the term agriculturally acceptable salt may include, for example, a metal salt, a salt with an organic base, a salt with an inorganic acid, a salt with an organic acid, a salt with a basic or acidic amino acid, and so forth. A suitable metal salt may include, for example, an alkali metal salt such as a sodium salt, a potassium salt, etc., an alkaline earth metal salt such as a calcium salt, a magnesium salt, a barium salt, etc., an aluminum salt, or the like. A salt with an organic base may include a salt with, for example, trimethylamine, triethylamine, pyridine, picoline, 2,6-lutidine, ethanolamine, diethanolamine, triethanolamine, cyclohexylamine, dicyclohexylamine, N,N-dibenzylethylenediamine, etc. A salt with an inorganic acid may include a salt with, for example, hydrochloric acid, hydrobromic acid, nitric acid, sulfuric acid, phosphoric acid, etc. A salt with an organic acid may include a salt with, for example, formic acid, acetic acid, trifluoroacetic acid, phthalic acid, fumaric acid, oxalic acid, tartaric acid, maleic acid, citric acid, succinic acid, methanesulfonic acid, benzenesulfonic acid, p-toluenesulfonic acid, etc. A salt with a basic amino acid may include a salt with, for example, arginine, lysine, ornithine, etc. A salt with an acidic amino acid may include a salt with, for example, aspartic acid, glutamic acid, etc.

Specifically, in the 2,5-diketopiperazine derivative represented by Chemical Formula 1, each of R¹ and R⁶, which are identical or different, may be a hydrogen atom, a methyl group, an ethyl group or a propyl group; and each of R², R³, R⁴ and R⁵, which are identical or different, may be a hydrogen atom, a methyl group, an ethyl group, a n-propyl group, an isopropyl group, a 2-methylethyl group, a 1-methylpropyl group, a 2-methylpropyl group, a hydroxymethyl group, a 1-hydroxyethyl group, a 2-hydroxyethyl group, an imidazol-4-yl-methyl group, a 2-methylthioethyl group, a benzyl group, a 4-hydroxybenzyl group, a phenethyl group, a mercaptomethyl group, a methylthiomethyl group, a 2-methylthioethyl group, a tritylthiomethyl group, a 2-tritylthioethyl group, a 2-ethoxycarbonylethyl group, a 2-methoxycarbonylethyl group, a methoxycarbonylmethyl group, a 2-methoxycarbonylethyl group, an ethoxycarbonylmethyl group, a 2-aminoethyl group, a carbamoylmethyl group, a 2-carbamoylethyl group, an acetylaminomethyl group, a 2-acetylaminoethyl group, a carboxymethyl group, a 2-carboxyethyl group, an imidazol-4-ylmethyl group, a 2-(imidazol-4-yl)ethyl group, a 3-guanidinopropyl, an indol-3-ylmethyl group or a 2-(indol-3-yl)ethyl group. One of R² and R³ may be linked to R¹ via -(CH₂)₃- to form a 5-membered ring or one of R⁴ and R⁵ may be linked to R⁶ via -(CH₂)₃- to form a 5-membered ring.

Specific 2,5-diketopiperazine derivatives for the use according to the invention are as follows:
3-hydroxymethyl-6-(2-methylpropyl)-2,5-diketopiperazine,
3-(2-methylpropyl)-2,5-diketopiperazine,
3-(1-hydroxyethyl)-6-(2-methylpropyl)-2,5-diketopiperazine,
3-isopxopyl-2,5-diketopiperazine,
3-(indol-3-ylmethyl)-2,5-diketopiperazine,
2,5-diketapiperazine,
3-methyl-2,5-diketopiperazine,
3,6-dimethyl-2,5-diketopiperazine,
3-methyl-6-(2-methylethyl)-2,5-diketopiperazine,
3-methyl-6-(4-hydroxybenzyl)-2,5-diketopiperazine,
3-(1-hydroxyethyl)-6-(2-mercaptomethyl)-2,5-diketopiperazine,
3-(1-hydroxyethyl)-6-(2-methylthiomethyl)-2,5-diketopiperazine,
3-(1-hydroxyethyl)-6-(2-tritylthiomethyl)-2,5-diketopiperazine,
3-(1-hydroxyethyl)-6-benzyl-2,5-diketopiperazine,
3-(1-hydroxyethyl)-6-(4-hydroxybenzyl)-2,5-diketopiperazine,
3-(1-hydroxyethyl)-6-(indol-3-ylmethyl)-2,5-diketopiperazine,
3-(2-methylpropyl)-6-(2-mercaptomethyl)-2,5-diketopiperazine,
3-(2-methylpropyl)-6-(2-methylthiomethyl)-2,5-diketopiperazine,
3-(2-methylpropyl)-6-(2-tritylthiomethyl)-2,5-diketopiperazine,
3-(2-methylpropyl)-6-benzyl-2,5-diketopiperazine,
3-(2-methylpropyl)-6-(4-hydroxybenzyl)-2,5-diketopiperazine,
3-(2-methylpropyl)-6-(indol-3-ylmethyl)-2,5-diketopiperazine,
3-(4-hydroxybenzyl)hexahydropyrrolo[1,2-a]pyrazin-1,A-dione,
3-benzylhexahydropyrrolo[1,2-a]pyrazin-1,4-dione,
3-methylhexahydropyrrolo[1,2-a]pyrazin-1,4-dione,
3-isopropylhexahydropyrrolo[1,2-a]pyrazin-1,4-dione,
3-(2-methylpropyl)hexahydropyrrolo[1,2-a]pyrazin-1,4-dione,
3-(1-methylpropyl)hexahydropyrrolo[1,2-a]pyrazin-1,4-dione, hexahydropyrrolo[1,2-a]pyrazin-1,4-dione,
3-(indol-3-ylmethyl)hexahydropyrrolo[1,2-a]pyrazin-1,4-dione, octahydropyrrolo[1,2-a:1',2'-d]pyrazin-5,10-dione,
3-hydroxymethylhexahydropyrrolo[1,2-a]pyrazin-1,4-dione,
3-(1-hydroxyethyl)hexahydropyrrolo[1,2-a]pyrazin-1,4-dione,
3-(2-carbamoylethyl)hexahydropyrrolo[1,2-a] pyrazin-1,4-dione,
3-(2-methylthioethyl)hexahydropyrrolo[1,2-a]pyrazin-1,4-dione,
3-carbamoylmethylhexahydropyrrolo[1,2-a]pyrazin-1,4-dione,
3-mercaptomethylhexahydropyrrolo[1,2-a]pyrazin-1,4-dione,
3-methylthiomethylhexahydropyrrolo[1,2-a]pyrazin-1,4-dione,
3-tritylthiomethylhexahydropyrrolo[1,2-a]pyrazin-1,4-dione,
3-(2-methoxycarbonylethyl)hexahydropyrrolo[1,2-a]pyrazin-1,4-dione,
3-(2-methoxycarbonylmethyl)hexahydropyrrolo[1,2-a]pyrazin-1,4-dione,
3-(2-aminoethyl)hexahydropyrrolo[1,2-a]pyrazin-1,4-dione,
3-(2-carboxyethyl)hexahydropyrrolo[1,2-a]pyrazin-1,4-dione,
3-(2-carboxymethyl)hexahydropyrrolo[1,2-a]pyrazin-1,4-dione,
3-(imidazol-4-ylmethyl)hexahydropyrrolo[1,2-a]pyrazin-1,4-dione and
3-(2-acetylaminoethyl)hexahydropyrrolo[1,2-a]pyrazin-1,4-dione.

Chemical structures of the 2,5-diketopiperazine derivatives represented by Chemical Formula 1 and synthesis methods thereof are already described in literatures [Akiyama et al, J. Chem. Soc., Perkin Trans. 1 1989, 235; Gordon et al, Bioorg. Med. Chem. Lett., 1995, 5, 47; Carlsson, A. C. Tetrahedron Lett. 2006, 47, 5199; Lopez-Cobenas, A. Synlett. 2005, 1158; Boehm et al, J. Org. Chem. 1986, 51, 2307]. Also, the inventors of the present invention have developed a method of directly synthesizing the 2,5-diketopiperazine derivative represented by Chemical Formula 1 via a one-pot process by cyclizing a linear dipeptide compound obtained by condensing two amino acid compounds in water solvent while heating at 80-180 °C [Kyung Seok Park, Surk Sik Moon, In Seok Hong, Korean Patent Application No. 2010-0039551 (Apr. 28,2010)].

The specific 2,5-diketopiperazine derivatives are useful as an active ingredient of an agricultural agent since it induces resistance of plants to soft rot, damping off, blight, withering, spot, mosaic disease, caused by bacteria, viruses or fungi. Specifically, it may be used for a dicotyledon such as tobacco, Chinese cabbage, red pepper, cucumber, potato, tomato.

The agricultural agent of the present invention may comprise 0.001-99 wt%, specifically 0.005-30 wt%, of the active ingredient and may further comprise an excipient as balance. The excipient may be a commonly used microbial agent, antibacterial agent, diluent or carrier. Further, in order to enhance efficacy or extend applications, commercially available or developed other microbicides, insecticides, herbicides, plant growth regulators or fertilizers may be added in addition to the active ingredient.

The excipient or diluent included in the agricultural agent of the present invention may be one commonly used in the agricultural industry. For example, oxides such as diatomite, slaked lime, etc., phosphates such as apatite, etc., sulfates such as gypsum, etc., mineral powders such as clay, kaolin, bentonite, acid clay, quartz, silica, etc. may be used. In addition to these solid carriers, fillers, anticoagulants, surfactants, emulsifiers, antiseptics, etc. may be included. The agricultural agent of the present invention may be formulated according to methods known in the art such that the active ingredient may be released immediately, in a sustained manner or in a delayed manner. It may be formulated into a wettable powder, a suspension, an emulsifiable concentrate, an emulsion, a microemulsion, a soluble concentrate, a dispersible concentrate, a water-dispersible granule, a granule, a dustable powder, a suspendable concentrate, a water-dispersible granule, a floating granule or a tablet by mixing the active ingredient with an additive commonly used for formulation such as a surfactant, a diluent, a dispersant, an adjuvant, etc.

The agricultural agent of the present invention may be applied to plants according to methods commonly employed in the art. For application to plants, it may be directly sprayed or applied to the leaves, stem, branches, root or seed of a plant or may be mixed in soil, earth or medium for raising seedlings. In case of plants growing in water, it may be treated on water to control diseases. Specifically, the agricultural agent may be treated through application, immersion, fumigation or spraying. For example, the agricultural agent may be sprayed onto soil or to the leaf, stem, seed, flower or fruit of plant. For application of the agricultural agent of the present invention to a plant, it may be diluted in water or a suitable medium.

Various experiments were carried out in order to investigate the ability of the agricultural agent of the present invention to induce resistance to plant diseases.

First, expression of the antifungal genes PR-1 and PDE1.2 was measured after treating thale cress with the 2,5-diketopiperazine compound of the present invention. Thale cress is frequently used in disease resistance studies because of abundant variants allowing understanding of disease resistance mechanisms. Remarkably increased expression of the PR-1 and PDE1.2 genes which inhibit plant diseases was observed in a test group treated with the 2,5-diketopiperazine compound of the present invention. Accordingly, it can be seen that the agricultural agent of the present invention provides a superior effect of preventing or treating plant diseases by inhibiting infection by and proliferation of pathogens. Indeed, when the leaves of tobacco with PR-1α promoter fused to the GUS gene were treated with the 2,5-diketopiperazine compound of the present invention, the PR-1α-GUS activity increased significantly as compared to a control group and, at the same time, anthracnose decreased by about 50% as compared to the control group. Further, in order to confirm the effect of the agricultural agent of the present invention of preventing plant diseases, a solution of the 2,5-diketopiperazine compound of the present invention was applied to tobacco, Chinese cabbage, cucumber, red pepper and Chinese cabbage and inhibitory effect on soft rot or anthracnose was evaluated. When the plants were treated with the 2,5₋diketopiperazine derivative of the present invention, inhibitory effect against plant diseases was remarkably improved as compared to an untreated control group or a group treated with a control agent (BTH). The effect of inhibiting plant diseases of the agricultural agent of the present invention will be described in more detail in the Examples section.

Accordingly, since the agricultural agent of the present invention is capable of inducing disease resistance, it is of great value as a next-generation agricultural agent.

### EXAMPLES

The present invention will be described in more detail through synthesis example and examples. However, the scope of this invention is not limited thereby.

### [Synthesis Example]

Representative Synthesis Example. Synthesis of 2,5-diketopiperazine derivatives

L- or D-Amino acid methyl ester hydrochloride (0.5 g, 3.6 mmol) was dissolved in 10 mL of dimethylformamide (DMF). Then, diisopropylethylamine (DIEA; 0.78 g, 6.0 mmol), various L- or D-N-Boc-amino acids (3.0 mmol) and *O*-(benzotriazol-1-yl)-*N,N,N,N*-tetramethyluronium hexafluorophosphate (HBTU; 1.37 g, 3.6 mmol) were added. After reaction at room temperature for 12 hours while stirring, DMF was removed from the reaction mixture under reduced pressure. The remaining mixture was diluted with ethyl acetate and washed with sodium bicarbonate and brine. The organic layer was concentrated under reduced pressure after removing water with anhydrous sodium sulfate. The concentrate was purified by silica column chromatography (EtOAc/hexane) to obtain a linear N-Boc-dipeptide. The linear *N*-Boc-dipeptide was added to a round-bottom flask and 20 mL of water added per 1 mmol of the linear *N*-Boc-dipeptide. Thereafter, the reaction vessel was fixed to a stainless-steel sterilization apparatus equipped with a pressure control valve. The sterilization apparatus was closed and the temperature of the reaction mixture was maintained at 130 °C for 4 hours. After stopping the reaction by lowering temperature, the pressure of the sterilization apparatus was decreased. After removing water under reduced pressure, the remaining concentrate was purified by silica column chromatography (MeOH/MC) to obtain a pure 2,5-diketopiperazine derivative. Nuclear magnetic resonance spectroscopic data of the prepared compounds are given below. They agree well to the data available from literatures.

The compounds prepared according to the method of Representative Synthesis Example are described in Table 1.

**Table 1**

| | | | | | | |
|---|---|---|---|---|---|---|
| 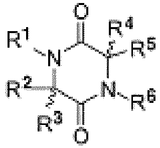 | | | | | | |

| Compound No. | R¹ | R² | R³ | R⁴ | R⁵ | R⁶ |
|---|---|---|---|---|---|---|
| 1 | H- | (CH₃)₂CHCH₂- | (S) H- | (S) H- | OHCH₂- | H- |
| 2 | H- | (CH₃)₂CHCH₂- | (S) H- | (S) H- | H- | H- |
| 3 | H- | (CH₃)₂CHCH₂- | (S) H- | (S) H- | CH₃CH(OH)- | H- |
| 4 | H- | (CH₃)₂CH- | (S) H- | (S) H- | H- | H- |
| 5 | H- | H- | H- | (S) H- | indol-3-yl-CH₂- | H- |
| 6 | H- | H- | H- | H- | H- | H- |
| 7 | H- | H- | H- | (S) H- | CH₃- | H- |
| 8 | H- | CH₃- | (S) H- | (S) H- | CH₃- | H- |
| 9 | H- | (CH₃)₂CH- | (S) H- | (S) H- | CH₃- | H- |
| 10 | H- | 4-HO-C₆H₄-CH₂- | (S) H- | (S) H- | CH₃- | H- |
| 11 | H- | HSCH₂- | (S) H- | (S) H- | OHCH₂- | H- |
| 12 | H- | CH₃SCH₂- | (S) H- | (S) H- | OHCH₂- | H- |
| 13 | H- | Ph₃CSCH₂- | (S) H- | (S) H- | OHCH₂- | H- |
| 14 | H- | C₆H₄-CH₂- | (S) H- | (S) H- | OHCH₂- | H- |
| 15 | H- | 4-HO-C₆H₄-CH₂- | (S) H- | (S) H- | OHCH₂- | H- |
| 16 | H- | indol-3-yl-CH₂- | (S) H- | (S) H- | OHCH₂- | H- |
| 17 | H- | HSCH₂- | (S) H- | (S) H- | CH₃CH(OH)- | H- |
| 18 | H- | CH₃SCH₂- | (S) H- | (S) H- | CH₃CH(OH)- | H- |
| 19 | H- | Ph₃CSCH₂- | (S) H- | (S) H- | CH₃CH(OH)- | H- |
| 20 | H- | C₆H₄-CH₂- | (S) H- | (S) H- | CH₃CH(OH)- | H- |
| 21 | H- | 4-HO-C₆H₄-CH₂- | (S) H- | (S) H- | CH₃CH(OH)- | H- |
| 22 | H- | indol-3-yl-CH₂- | (S) H- | (S) H- | CH₃CH(OH)- | H- |
| 23 | H- | 4-HO-C₆H₄-CH₂- | (S) H- | (S) H- | -CH₂CH₂CH₂- | |
| 24 | H- | C₆H₄-CH₂- | (S) H- | (S) H- | -CH₂CH₂CH₂- | |
| 25 | H- | CH₃- | (S) H- | (S) H- | -CH₂CH₂CH₂- | |
| 26 | H- | (CH₃)₂CH- | (S) H- | (S) H- | -CH₂CH₂CH₂- | |
| 27 | H- | (CH₃)₂CHCH₂- | (S) H- | (S) H- | -CH₂CH₂CH₂- | |
| 28 | H- | CH₃CH₂(CH₃)CH- | (S) H- | (S) H- | -CH₂CH₂CH₂- | |
| 29 | H- | H- | (S) H- | (S) H- | -CH₂CH₂CH₂- | |
| 30 | H- | indol-3-yl-CH₂- | (S) H- | (S) H- | -CH₂CH₂CH₂- | |
| 31 | -CH₂CH₂CH₂- | | (S) H- | (S) H- | -CH₂CH₂CH₂- | |
| 32 | H- | HOCH₂- | (S) H- | (S) H- | -CH₂CH₂CH₂- | |
| 33 | H- | CH₃CH(OH)- | (S) H- | (S) H- | -CH₂CH₂CH₂- | |
| 34 | H- | C₆H₄-CH₂- | (S) H- | (R) H- | -CH₂CH₂CH₂- | |
| 35 | H- | 4-HO-C₆H₄-CH₂- | (S) H- | (R) H- | -CH₂CH₂CH₂- | |
| 36 | H- | (CH₃)₂CH- | (S) H- | (R) H- | -CH₂CH₂CH₂- | |
| 37 | H- | NH₂COCH₂CH₂- | (S) H- | (R) H- | -CH₂CH₂CH₂- | |
| 38 | H- | CH₃- | (S) H- | (R) H- | -CH₂CH₂CH₂- | |
| 39 | H- | CH₃SCH₂CH₂- | (S) H- | (R) H- | -CH₂CH₂CH₂- | |
| 40 | H- | CH₃SCH₂CH₂- | (S) H- | (R) H- | -CH₂CH₂CH₂- | |
| 41 | H- | CH₃CH(OH)- | (S) H- | (R) H- | -CH₂CH₂CH₂- | |
| 42 | H- | HOCH₂- | (S) H- | (R) H- | -CH₂CH₂CH₂- | |
| 43 | H- | CH₃CH₂(CH₃)CH- | (S) H- | (R) H- | -CH₂CH₂CH₂- | |
| 44 | H- | (CH₃)₂CHCH₂- | (S) H- | (R) H- | -CH₂CH₂CH₂- | |
| 45 | -CH₂CH₂CH₂- | | (S) H- | (R) H- | -CH₂CH₂CH₂- | |
| 46 | H- | H- | H- | (R) H- | -CH₂CH₂CH₂- | |
| 47 | H- | 4-HO-C₆H₄-CH₂- | (R) H- | (S) H- | -CH₂CH₂CH₂- | |
| 48 | H- | (CH₃)₂CHCH₂- | (R) H- | (S) H- | -CH₂CH₂CH₂- | |
| 49 | H- | (CH₃)₂CH- | (R) H- | (S) H- | -CH₂CH₂CH₂- | |
| 50 | H- | CH₃- | (R) H- | (S) H- | -CH₂CH₂CH₂- | |
| 51 | H- | indol-3-yl-CH₂- | (R) H- | (S) H- | -CH₂CH₂CH₂- | |
| 52 | H- | HOCH₂- | (R) H- | (S) H- | -CH₂CH₂CH₂- | |
| 53 | H- | CH₃- | (R) H- | (R) H- | -CH₂CH₂CH₂- | |
| 54 | H- | indol-3-yl-CH₂- | (R) H- | (R) H- | -CH₂CH₂CH₂- | |
| 55 | H- | HOCH₂- | (R) H- | (R) H- | -CH₂CH₂CH₂- | |
| 56 | H- | CH₃CH(OH)- | (R) H- | (S) H- | -CH₂CH₂CH₂- | |
| 57 | H- | CH₃SCH₂CH₂- | (R) H- | (S) H- | -CH₂CH₂CH₂- | |
| 58 | H- | C₆H₄-CH₂- | (R) H- | (S) H- | -CH₂CH₂CH₂- | |
| 59 | H- | NH₂COCH₂CH₂- | (R) H- | (S) H- | -CH₂CH₂CH₂- | |
| 60 | H- | CH₃CH₂(CH₃)CH- | (R) H- | (S) H- | -CH₂CH₂CH₂- | |
| 61 | H- | ((CH₃)₂CHCH₂- | (R) H- | (R) H- | -CH₂CH₂CH₂- | |
| 62 | H- | (CH₃)₂CH₂- | (R) H- | (R) H- | -CH₂CH₂CH₂- | |
| 63 | H- | C₆H₄-CH₂- | (R) H- | (R) H- | -CH₂CH₂CH₂- | |
| 64 | H- | 4-HO-C₆H₄-CH₂- | (R) H- | (R) H- | -CH₂CH₂CH₂- | |
| 65 | H- | CH₃SCH₂CH₂- | (R) H- | (R) H- | -CH₂CH₂CH₂- | |
| 66 | H- | CH₃CH(OH)- | (R) H- | (R) H- | -CH₂CH₂CH₂- | |
| 67 | H- | CH₃CH₂(CH₃)CH- | (R) H- | (R) H- | -CH₂CH₂CH₂- | |
| 68 | H- | NH₂COCH₂- | (S) H- | (S) H- | -CH₂CH₂CH₂- | |
| 69 | H- | HSCH₂- | (S) H- | (S) H- | -CH₂CH₂CH₂- | |
| 70 | H- | CH₃SCH₂- | (S) H- | (S) H- | -CH₂CH₂CH₂- | |
| 71 | H- | Ph₃CSCH₂- | (S) H- | (S) H- | -CH₂CH₂CH₂- | |
| 72 | H- | CH₃OOCCH₂CH₂- | (S) H- | (S) H- | -CH₂CH₂CH₂- | |
| 73 | H- | CH₃OOCCH₂- | (S) H- | (S) H- | -CH₂CH₂CH₂- | |
| 74 | H- | NH₂(CH₂)₂- | (S) H- | (S) H- | -CH₂CH₂CH₂- | |
| 75 | H- | HOOCCH₂CH₂- | (S) H- | (S) H- | -CH₂CH₂CH₂- | |
| 76 | H- | HOOCCH₂- | (S) H- | (S) H- | -CH₂CH₂CH₂- | |
| 77 | H- | imidazol-4-yl-CH₂- | (S) H- | (S) H- | -CH₂CH₂CH₂- | |
| 78 | H- | AcNH(CH₂)₂- | (S) H- | (S) H- | -CH₂CH₂CH₂- | |
| 79 | H- | NH₂CO(CH₂)₂- | (S) H- | (S) H- | -CH₂CH₂CH₂- | |
| 80 | H- | 4-HO-C₆H₄-CH₂- | (S) H- | (R) H- | -CH₂CH₂CH₂- | |

Compound No. 1. white solid, ¹H-NMR (CD₃OD-*d*₄, 400 MHz) δ 0.95 (d, *J* = 6 Hz, 3H), 0.95 (d, *J* = 6 Hz, 3H), 1.80 (m, 2H), 1.85 (m, 1H), 3.67 (dd, *J* = 4.4 Hz and 12.8 Hz, 1H), 3.92 (m, 1H, overlapped), 3.90 (t, *J* = 3.6 Hz, 1H), 3.92 (m, 1H), ¹³C NMR (CD₃OD-*d*₄,100 MHz) δ 20.6, 22.4, 23.8, 45.0, 53.4, 57.8, 62.7, 167.6, 170.2.
Compound No. 2. colorless crystal, mp 241-244 °C, ¹H NMR (DMSO-*d*₆, 400 MHz) δ 8.21 (1H, s), 7.95 (1H, s), 3.78 (2H, d), 3.65-3.56 (2H, m), 1.76-1.72 (1H, m), 1.52-1.48 (1H, t), 0.87-0.83 (6H, d), ¹³C NMR (DMSO-*d*₆, 100 MHz) δ 169.3, 166.9, 53.5, 44.9, 42.8, 24.2, 23.5, 22.4.
Compound No. 3. white solid, ¹H-NMR (CD₃OD-*d*₄, 400 MHz) δ 0.93 (d, *J* = 6.4 Hz, 3H), 0.94 (d, *J* = 6.4 Hz, 3H), 1.23 (d, *J* = 6.8 Hz, 3H), 1.72 (m, 1H), 1.85 (m,1H), 1.86 (m, 1H overlapped), 3.71 (d, *J* = 2.4 Hz, 1H), 3.87 (dd, *J* = 4.0 Hz and 9.0 Hz, 1H), 4.17 (dq, *J* = 6.4 Hz, 1H), ¹³C NMR (CD₃OD-*d*₄, 100 MHz) δ 18.5, 20.2, 22.2, 23.6, 45.0, 53.2, 60.8, 67.3, 168.0, 170.4.
Compound No. 4. ¹H-NMR (CD₃OD-*d*₄, 400 MHz) δ 0.95 (d, *J* = 6.4 Hz, 3H), 0.97 (d, *J* = 6.8 Hz, 3H), 1.65 (m, 1H), 1.67 (m, 1H), 1.80 (m, 1H), 3.82 (d, *J* = 17.6 Hz, 1H), 3.88 (m, 1H), 4.00 (d, *J* = 17.6 Hz, 1H), ¹³C NMR (CD₃OD-*d*₄, 100 MHz) δ 20.6, 22.0, 23.8, 42.4, 43.8, 53.3, 167.4, 170.1.
Compound No. 5. white solid, mp 280-284 °C (dec), ¹H NMR (DMSO-*d*₆, 400 MHz) δ 10.92 (1H, s), 8.09 (1H, s), 7.75 (1H, s), 7.53 (1H, d), 7.31 (1H, d), 7.04 (2H, m), 6.93 (1H, t), 4.00 (1H, d), 3.32-3.21 (2H, dd), 2.98 (1H, dd), 2.76 (1H, d), ¹³C NMR (DMSO-*d*₆, 100 MHz) δ 168.6, 166.3, 136.6, 128.1, 125.2, 121.5, 119.3, 119.1, 111.8, 109.0, 56.1, 44.5, 29.8.
Compound No. 6. white solid, mp 210-215 °C (dec), ¹H NMR (DMSO-*d*₆, 400 MHz) δ 7.98 (2H, s), 3.68 (4H, d), ¹³C NMR (DMSO-*d*₆, 100 MHz) δ 166.7 (2C), 45.0 (2C).
Compound No. 7. white solid, mp 210-212 °C (dec), ¹H NMR (DMSO-*d*₆, 400 MHz) δ 8.13 (1H, s), 7.94 (1H, s), 3.81 (1H, q), 3.70 (1H, d), 1.22 (3H, d), ¹³C NMR (DMSO-*d*₆, 100 MHz) δ 169.5, 166.9, 50.3, 45.1, 19.3.
Compound No. 8. white crystal, mp 245-247 °C (dec), ¹H NMR (DMSO-*d*₆, 400 MHz) δ 8.06 (2H, s), 3.85 (2H, q), 1.21 (6H, d);¹³C NMR (DMSO-*d*₆, 100 MHz) δ 169.7 (2C), 50.4 (2C), 19.1 (2C).
Compound No. 9. white solid, mp 220-222 °C (dec), ¹H NMR (DMSO-*d*₆, 400 MHz) δ 8.10 (1H, s), 7.96 (1H, s), 3.83 (1H, q), 3.65 (1H, s), 2.14-2.10 (1H, m), 1.24 (3H, d), 0.91 (3H, d), 0.80 (3H, d), ¹³C NMR (DMSO-*d*₆,100 MHz) δ 169.3, 167.3, 60.0, 50.3, 31.7, 20.7, 19.1, 17.5.
Compound No. 10. white solid, mp 276-280 °C (dec), ¹H NMR (DMSO-*d*₆, 400 MHz) δ 9.23 (1H, s), 8.01 (1H, s), 7.95 (1H, s), 6.89 (2H, d), 6.62 (2H, d), 4.05 (1H, s), 3.56 (1H, q), 3.01-2.96 (1H, dd), 2.73-2.68 (1H, dd), 0.50 (3H, d), ¹³C NMR (DMSO-*d*₆, 100 MHz) δ 168.3, 166.6, 156.8, 131.9 (2C), 126.5, 115.5 (2C), 56.2, 50.4, 38.2, 20.4.
Compound No. 23. white solid, mp 147-149 °C, R_{f} 0.16 (0.5:9.5 MeOH/MC), ¹H NMR (CDCl₃, 400 MHz) δ 7.05 (2H, d), 6.77 (2H, d), 5.72 (1H, s), 4.21 (1H, dd), 4.08 (1H, t), 3.66-3.59 (1H, m), 3.56-3.51 (1H, m), 3.44-3.39 (1H, m), 2.75 (1H, dd), 2.29 (1H, m), 2.02-1.96 (1H, m), 1.94-1.83 (2H, m), ¹³C NMR (CDCl₃, 100 MHz) δ 169.8, 165.3, 155.6, 130.5 (2C), 127.4, 116.3 (2C), 59.3, 56.4, 45.6, 36.1, 28.5, 22.7; HR-TOF-ESI-MS (*m*/*z*): C₁₄H₁₆N₂O₃(M+H)⁺ theoretical 261.1161, measured 261.1226; [α]_{D} = -71.3° (c 0.5, MeOH).
Compound No. 24. white solid, R_{f} 0.33 (MeOH:MC/0.5:9.5), mp 133-135 °C, ¹H NMR (CDCl₃, 400 MHz) δ 7.36-7.21 (5H, m), 5.62 (1H, s), 4.25 (1H, dd), 4.05 (1H, t), 3.66-3.54 (3H, m), 2.74 (1H, dd), 2.36-2.30 (1H, m), 2.05-1.87 (3H, m), ¹³C NMR (CDCl₃, 100 MHz) δ 169.6, 165.2, 136.1, 129.5 (2C), 129.3 (2C), 127.78, 59.3, 56.3, 45.6, 36.9, 28.5, 22.7.
Compound No. 25. white solid, ¹H NMR (400 MHz, methanol-*d*₄) δ 4.25 (1H, dd, *J* = 9.2, 1.6, Hz), 4.17 (1H, q, *J* = 11.6, 6.0 Hz), 3.51 (2H, m), 2.30 (1H, m), 1.96-2.05 (3H, m), 1.37 (3H, d, *J* = 6.8 Hz).
Compound No. 26. white solid, mp 177-179 °C, ¹H NMR (400 MHz, methanol-*d*₄) δ 4.19 (1H, br t, *J* = 8.0 Hz), 4.03 (1H, brs, H3), 3.46-3.59 (2H, m), 2.48 (1H, m), 2.31 (1H, m), 1.95 (1H, m), 1.89∼2.04 (2H, m), 1.09 (3H, d, *J* = 7.2 Hz), 0.93 (3H, d, *J* = 7.2 Hz), ¹³C NMR (100 MHz, methanol-*d*₄) δ 171.4, 166.4, 60.3, 58.8, 45.0, 28.7, 28.3, 22.1, 17.6, 15.5.
Compound No. 27. white solid, mp 161-163 °C, ¹H NMR (400 MHz, methanol-*d*₄) δ 4.25 (1H, br t, *J* = 8.0 Hz), 4.12 (1H, m), 3.51 (2H, m), 2.30 (1H, m), 2.01 (1H, m), 1.89-2.01 (2H, m), 1.88 (1H, m), 1.85 (1H, m), 0.96 (3H, d, *J* = 6.8 Hz), 0.95 (3H, d, *J* = 6.8 Hz), ¹³C NMR (100 MHz, methanol-*d*₄) δ 171.6, 167.7, 59.1, 53.4, 45.2, 38.2, 27.9, 24.6, 22.5, 22.1, 22.0.
Compound No. 28. mp 104-105 °C, ¹H NMR (CDCl₃, 400 MHz) δ 6.68(1H, s), 4.01 1H, t), 3.93 1H, s), 3.92-3.50 2H, m), 2.36-2.25 2H, m), 2.02-1.93 2H, m), 1.88-1.85 1H, m), 1.40-1.36 1H, m), 1.21-1.15 1H, m), 1.02 3H, d), 0.88 3H, t), ¹³C NMR (CDCl₃, 100 MHz) δ 170.4, 165.3, 60.7, 58.9, 45.3, 35.5, 28.7, 24.2, 22.5, 15.9, 12.3.
Compound No. 29. white solid, mp 152-156 °C, ¹H NMR (CDCl₃, 400 MHz) δ 7.28 (1H, s), 4.05 (1H, t), 4.03 (1H, s), 3.84 (2H, dd), 3.61-3.50 (2H, m), 2.36-2.31 (1H, m), 2.06-1.98 (2H, m), 1.91-1.87 (1H, m), ¹³C NMR (CDCl₃, 100 MHz) δ 170.4, 163.8, 58.7, 46.8, 45.5, 28.6, 22.6.
Compound No. 30. white solid, R_{f} 0.16 (0.5:9.5 MeOH/MC), ¹H NMR (CDCl₃, 400 MHz) δ 7.05 (2H, d, *J* = 8.4 Hz), 6.77 (2H, d, *J* = 8.4 Hz), 5.72 (1H, s), 4.21 (1H, d, *J* = 6.4 Hz), 4.08 (1H, t, *J* = 6.8 Hz), 3.66-3.59 (1H, m), 3.56-3.51 (1H, m), 3.44-3.39 (1H, m), 2.75 (1H, dd, *J* = 10.0, 14.4 Hz), 2.29 (1H, m), 2.02-1.96 (1H, m), 1.94-1.83 (2H, m), ¹³C NMR (CDCl₃, 100 MHz) δ 169.8, 165.3, 155.6, 130.5 (2C), 127.4, 116.3 (2C), 59.3, 56.4, 45.6, 36.1, 28.5, 22.7; ESI-MS (*m*/*z*): C₁₄H₁₆N₂O₃ (M+H)⁺ theoretical 261.1161; measured 261.1226; [α]_{D} =-71.3° (c 0.5, MeOH).
Compound No. 31. white solid, mp 140-141 °C, ¹H NMR (CDCl₃, 400 MHz) δ 4.10 (2H, t), 3.45 (4H, dd), 2.28-2.22 (2H, m), 2.14-2.09 (2H, m), 1.94-1.92 (2H, m), 1.84-1.89 (2H, m), ¹³C NMR (CDCl₃, 100 MHz) δ 166.6 (2C), 60.7 (2C), 45.3 (2C), 27.8 (2C), 23.5 (2C).
Compound No. 32. white solid, mp 152-156 °C, ¹H NMR (CDCl₃, 400 MHz) δ 7.40 (1H, s), 4.10 (2H, t), 3.97 (2H, dd), 3.60-3.52 (2H, m), 2.36-2.30 (1H, m), 2.06-1.98 (2H, m), 1.90-1.86 (1H, m), ¹³C NMR (CDCl₃, 100 MHz) δ 170.5, 165.2, 61.2, 59.2, 56.7, 45.5, 28.4, 22.7.
Compound No. 33. white solid, ¹H NMR (CDCl₃, 400 MHz) δ 7.09 (1H, s), 4.27 (1H, dd), 4.01 (2H, t), 3.88 (1H, d), 3.57-3.50 (1H, m), 3.47-3.41 (1H, m), 2.29-2.23 (1H, m), 2.04-1.90 (3H, m), 1.86-1.81 (1H, m), 1.27 (3H, d), ¹³C NMR (CDCl₃, 100 MHz) δ 170.7, 165.5, 65.8, 59.6, 59.1, 45.5, 28.2, 22.8.
Compound No. 34. white solid, mp 148-151 °C, ¹H NMR (400 MHz, methanol-*d*₄) δ 7.28-7.31 (3H, m), 7.18 (2H, dd, *J* = 7.2, 2.4 Hz), 4.20 (1H, t, *J* = 4.8 Hz), 3.53 (1H, dt, *J* = 12.0, 8.4 Hz), 3.27-3.33 (1H, m), 3.19 (1H, dd, *J* = 14.0, 4.8 Hz), 2.99 (1H, dd, *J* = 14.0, 4.8 Hz), 2.60 (1H, t, *J* = 6.4 Hz), 2.02 (1H, m), 1.90 (1H, m), 1.67 (1H, m), 1.61 (1H, m), ¹³C NMR (100 MHz, methanol-*d*₄) δ 170.1, 166.2, 135.5, 130.1, 128.5, 127.3, 58.6, 57.9, 45.0, 39.8, 28.6, 21.3.
Compound No. 35. white solid, mp 144-146 °C, ¹H NMR (400 MHz, methanol-*d*₄) δ 6.97 (2H, d, *J* = 8.4 Hz), 6.71 (2H, d, *J* = 8.4 Hz), 4.14 (1H, t, *J* = 4.4 Hz), 3.53 (1H, dt, *J* = 12.0, 8.4 Hz), 3.30 (1H, m), 3.10 (1H, dd, *J* = 14.0, 4.0 Hz), 2.87 (1H, dd, *J* = 14.0, 4.0 Hz), 2.60 (1H, dd, *J* = 10.4, 6.4 Hz), 2.05 (1H, m), 1.90 (1H, m), 1.65 (1H, m), 1.64 (1H, m), ¹³C NMR (100 MHz, methanol-*d*₄) δ 170.2, 166.4, 157.1, 131.1, 125.8, 115.2, 58.7, 58.0, 44.9, 39.0, 28.7, 21.3.
Compound No. 36. ¹H NMR (400 MHz, methanol-*d*₄) δ 4.23 (1H, dd, *J* = 10, 6.4 Hz), 3.64-3.57 (2H, m), 3.48 (H, m), 2.34 (1H, m), 2.13 (1H, sext, *J* = 6.6 Hz), 2.01 (1H, m), 1.86-1.90 (2H, m), 1.01 (3H, d, *J* = 7.2 Hz), 0.98 (3H, d, *J* = 7.2 Hz).
Compound No. 38. ¹H NMR (400 MHz, methanol-*d*₄) δ 4.26 (1H, dd, *J* = 10, 6.0 Hz), 3.90 (1H, ddd, *J* = 17.6, 6.4, 1.2), 3.58 (1H, m), 3.48 (1H, m), 2.34 (1H, m), 2.01 (1H, m), 1.88-1.94 (2H, m), 1.43 (3H, d, *J* = 7.2 Hz), ¹³C NMR (100 MHz, methanol-*d*₄) δ 170.0, 168.2, 59.4, 54.6, 46.8, 30.1, 23.1, 20.0.
Compound No. 39. white solid, ¹H NMR (CDCl₃, 400 MHz) δ 7.33 (1H, s), 4.20 (1H, t, *J* = 5.6 Hz), 4.10 (1H, t, *J* = 8.2 Hz), 3.61-3.48 (2H, m), 2.69 (2H, t, *J* = 7.0 Hz), 2.41-2.28 (2H, m), 2.10 (3H, s), 2.07-1.93 (3H, m), 1.91-1.84 (1H, m).
Compound No. 40. white solid, ¹H NMR (CDCl₃, 400 MHz) δ 7.55 (1H, s), 4.05 (2H, m), 3.63-3.56 (1H, m), 3.50-3.44 (1H, m), 2.65-2.51 (2H, m), 2.38-2.31 (1H, m), 2.05 (3H, s), 2.01-1.80 (4H, m), 1.80-1.70 (1H, m).
Compound No. 41. white solid, ¹H NMR (400 MHz, methanol-*d*₄) δ 4.29 (1H, dd, *J* = 10.8, 6.4 Hz), 4.18 (1H, ddd, *J* = 12.8, 8.8, 2.0), 3.66 (1H, dd, *J* = 2.0, 0.8), 3.60-3.48 (2H, m), 2.32 (1H, m), 2.01 (1H, m), 1.80-1.94 (2H, m), 1.23 (3H, d, *J* = 7.2 Hz), ¹³C NMR (100 MHz, methanol-*d*₄) δ 172.6, 168.0, 70.6, 64.7, 60.2, 46.6, 30.2, 23.0, 20.1.
Compound No. 43. ¹H NMR (400 MHz, methanol-*d*₄) δ 4.23 (1H, dd, *J* = 9.8, 6.8 Hz), 3.67 (1H, d, *J* = 6.4 Hz), 3.60 (1H, m), 3.48 (H, m), 2.34 (1H, m), 2.0 (1H, m), 1.86-1.90 (3H, m), 1.59 (1H, m), 1.22, (1H, m), 0.99 (3H, d, *J* = 7.2 Hz), 0.94 (3H, d, *J* = 7.6 Hz), ¹³C NMR (100 MHz, methanol-*d*₄) δ 170.4, 167.7, 62.3, 58.5, 45.6, 39.8, 29.1, 24.8, 21.7, 14.5, 10.4.
Compound No. 44. ¹H NMR (400 MHz, methanol-*d*₄) δ 4.25 (1H, dd *J* = 9.2, 6.8 Hz), 3.84 (1H, dd, *J* = 9.6, 5.2 Hz), 3.48-3.56 (2H, m), 2.33 (1H, m), 1.90-2.04 (3H, m), 1.76 (1H, m), 1.67, (1H, ddd, *J* = 14.8, 10.0, 6.0 Hz), 1.56 (1H, ddd, *J* = 13.6, 8.4, 5.2 Hz), 0.98 (3H, d, *J* = 6.4 Hz), 0.96 (3H, d, *J* = 6.4 Hz), ¹³C NMR (100 MHz, methanol-*d*₄) δ 170.4, 167.9, 58.1, 55.9, 45.5, 42.3, 28.7, 24.3, 21.9, 22.1, 20.7.
Compound No. 45. white solid, ¹H NMR (CDCl₃, 400MHz) δ 4.01-3.96 (4H, m), 3.34-3.27 (2H, m), 2.46-2.39 (2H, m), 2.07-1.98 (2H, m), 1.94-1.86 (2H, m), 1.81-1.73 (2H, m).
Compound No. 46. white solid, ¹H NMR (CDCl₃, 400 MHz) δ 7.30 (1H, s), 4.09 (1H, t), 4.05 (1H, s), 3.91(2H, dd), 3.63-3.52 (2H, m), 2.39-2.33 (1H, m), 2.08-2.00 (2H, m), 1.95-1.87 (1H, m), ¹³C NMR (CDCl₃, 100 MHz) δ170.1, 163.6, 58.5, 46.5, 45.2, 28.4, 22.3.
Compound No. 47. white solid, ¹H NMR (DMSO-*d*₆,, 400 MHz) δ 9.29 (1H, s), 8.10 (1H, d), 6.89 (2H, d), 6.65 (2H, d), 3.90 (1H, dd), 3.42 (1H, dd), 3.20-3.14 (1H, m), 2.92-2.82 (2H, m), 2.76 (1H, dd), 1.98-1.91 (2H, m), 1.78-1.72 (1H, m), 1.59-1.54 (1H, m), ¹³C NMR (DMSO-*d*₆, 100 MHz) δ168.3, 164.8, 156.3, 130.7 (2C), 125.8, 115.0 (2C), 58.1, 57.1, 44.5, 38.6, 28.5, 21.2.
Compound No. 48. white solid, ¹H NMR (CDCl₃, 400 MHz) δ 7.44 (1H, s), 4.05 (1H, dd, *J* = 8.8, 6.4 Hz), 3.91-3.86 (1H, m), 3.63-3.56 (1H, m), 3.53-3.45 (1H, m), 2.35-2.31 (1H, m), 2.00-1.93 (2H, m), 1.89-1.71 (2H, m), 1.64-1.59 (2H, m), 0.97 (3H, d, *J* = 6.8 Hz), 0.94 (3H, d, *J* = 6.8 Hz), ¹³C NMR (CDCl₃, 100 MHz) δ 170.0, 166.7, 58.2, 56.4, 45.7, 42.8, 29.1, 24.6, 23.2, 22.4, 21.6.
Compound No. 49. white solid, ¹H NMR (CDCl₃, 400 MHz) δ 6.98 (1H, s), 4.09 (1H, t, *J* = 6.8 Hz), 3.73-3.62 (1H, m), 3.53-3.47 (1H, m), 2.41-2.35 (1H, m), 2.23-2.16 (1H, m), 2.04-1.96 (1H, m), 1.94-1.82 (2H, m), 1.03 (3H, d, *J* = 6.8 Hz), 0.88 (3H, d, *J* = 6.8 Hz.
Compound No. 50. white solid, ¹H NMR (CDCl₃, 400 MHz) δ 6.99 (1H, s), 4.11-4.08 (2H, m), 3.61-3.47 (2H, m), 2.35-2.27 (1H, m), 2.13-1.82 (3H, m), 1.45 (3H, d, *J* = 6.8 Hz), ¹³C NMR (CDCl₃, 100 MHz) δ 170.8, 166.6, 59.4, 51.3, 45.6, 28.3, 22.9, 16.0.
Compound No. 53. white solid, ¹H NMR (400 MHz, methanol-*d*₄) δ 4.25 (1H, dd, *J* = 9.2, 1.6, Hz), 4.17 (1H, q, *J* = 11.6, 6.0 Hz), 3.51 (2H, m, H9), 2.30 (1H, m), 1.96-2.05 (3H, m), 1.37 (3H, d, *J* = 6.8 Hz).
Compound No. 54. white solid, ¹H NMR (CDCl₃, 400 MHz) δ 7.05 (2H, d, *J* = 8.4 Hz), 6.77 (2H, d, *J* = 8.4 Hz), 5.72 (1H, s), 4.21 (1H, d, *J* = 6.4 Hz), 4.08 (1H, t, *J* = 6.8 Hz), 3.66-3.59 (1H, m), 3.56-3.51 (1H, m), 3.44-3.39 (1H, m), 2.75 (1H, dd, *J* = 10.0, 14.4 Hz), 2.29 (1H, m), 2.02-1.96 (1H, m), 1.94-1.83 (2H, m), ¹³C NMR (CDCl₃, 100 MHz) δ 169.8,165.3,155.6,130.5 (2C), 127.4, 116.3 (2C), 59.3, 56.4, 45.6, 36.1, 28.5, 22.7.
Compound No. 55. white solid, ¹H NMR (CDCl₃, 400 MHz) δ 7.40 (1H, s), 4.10 (2H, t), 3.97 (2H, dd), 3.60-3.52 (2H, m), 2.36-2.30 (1H, m), 2.06-1.98 (2H, m), 1.90-1.86 (1H, m), ¹³C NMR (CDCl₃, 100 MHz) δ170.5, 165.2, 61.2, 59.2, 56.7, 45.5, 28.4, 22.7.
Compound No. 56. white solid, ¹H NMR (DMSO-*d*₆, 400 MHz) δ 8.18 (1H, d), 5.04 (1H, d), 4.12 (1H, dd), 3.98-3.95 (1H, m), 3.43-3.28 (3H, m), 2.13-2.08 (1H, m), 1.85-1.79 (1H, m), 1.77-1.63 (2H, m), 1.07 (3H, d), ¹³C NMR (DMSO-*d*₆, 100 MHz)δ 169.1, 164.8, 67.8, 62.6, 57.6, 44.4, 28.3, 21.1, 19.8.
Compound No. 57. white solid, ¹H NMR (CDCl₃, 400 MHz) δ 7.55 (1H, s), 4.05 (2H, m), 3.63-3.56 (1H, m), 3.50-3.44 (1H, m), 2.65-2.51 (2H, m), 2.38-2.31 (1H, m), 2.05 (3H, s), 2.01-1.80 (4H, m), 1.80-1.70 (1H, m).
Compound No. 58. white solid, ¹H NMR (400 MHz, methanol-*d*₄) δ 7.28-7.31 (3H, m), 7.18 (2H, dd, *J* = 7.2, 2.4 Hz), 4.20 (1H, t, *J* = 4.8 Hz), 3.53 (1H, dt, *J* = 12.0, 8.4 Hz), 3.27-3.33 (1H, m), 3.19 (1H, dd, *J* = 14.0, 4.8 Hz), 2.99 (1H, dd, *J* = 14.0, 4.8 Hz), 2.60 (1H, t, *J* = 6.4 Hz), 2.02 (1H, m), 1.90 (1H, m), 1.67 (1H, m), 1.61 (1H, m), ¹³C NMR (100 MHz, methanol-*d*₄) δ 170.1, 166.2, 135.5, 130.1, 128.5, 127.3, 58.6, 57.9, 45.0, 39.8, 28.6, 21.3.
Compound No. 59. white solid, ¹H NMR (CDCl₃, 400 MHz) δ 7.1 (1H, s), 4.08 (1H, dd), 3.77 (1H, dd), 3.70-3.63 (1H, m), 3.51-3.46 (1H, m), 2.40-2.34 (1H, m), 2.03-1.82 (4H, m), 1.59-1.50 (1H, m), 1.25-1.18 (1H, m), 1.00 (3H, d), 0.92 (3H, t), ¹³C NMR (CDCl₃, 100 MHz) δ 169.8, 165.5, 63.0, 58.5, 45.8, 39.8, 29.5, 24.7, 22.1, 15.4, 11.5.
Compound No. 61. white solid, ¹H NMR (CDCl₃, 400 MHz) δ 6.56 (1H, s), 4.10 (1H, t, *J* = 8.4 Hz), 3.99 (1H, dd, *J* = 9.2, 3.6 Hz), 3.60-3.47 (2H, m), 2.32-2.27 (1H, m), 2.11-1.94 (3H, m), 1.90-1.85 (1H, m), 1.82-1.73 (1H, m), 1.53-1.46 (1H, m), 0.97 (3H, d, *J* = 6.8 Hz), 0.92 (3H, d, *J* = 6.8 Hz), ¹³C NMR (CDCl₃, 100MHz) δ 170.6, 166.5, 59.2, 53.6, 45.6, 38.7, 28.2, 24.8, 23.4, 22.9, 21.4.
Compound No. 62. white solid, ¹H NMR (CDCl₃, 400 MHz) δ 6.17 (1H, s), 4.08 (1H, t, *J* = 8.0 Hz), 3.92 (1H, s), 3.66-3.59 (1H, m), 3.55 (1H, m), 2.65-2.58 (1H, m), 2.39-2.33 (1H, m), 2.08-1.98 (2H, m), 1.93-1.83 (1H, m), 1.07 (3H, d, *J* = 7.2 Hz), 0.88 (3H, d, *J* = 7.2 Hz).
Compound No. 63. white solid, ¹H NMR (CDCl₃, 400 MHz) δ 7.30 (1H, s), 4.29 (1H, dd), 4.05 (2H, t), 3.89 (1H, d), 3.57-3.50 (1H, m), 3.47-3.41 (1H, m), 2.29-2.23 (1H, m), 2.04-1.90 (3H, m), 1.86-1.81 (1H, m), 1.28 (3H, d), ¹³C NMR (CDCl₃, 100 MHz) δ 170.5, 165.6, 65.7, 59.7, 59.1, 45.4, 28.2, 22.7, 19.4.
Compound No. 64. white solid, ¹H NMR (CDCl₃, 400 MHz) δ 6.57 (1H, s), 4.04 (1H, t), 3.93 (1H, s), 3.63-3.47 (2H, m), 2.35-2.24 (2H, m), 2.02-1.93 (2H, m), 1.88-1.85 (1H, m), 1.43-1.35 (1H, m), 1.22-1.15 (1H, m), 1.04 (3H, d), 0.90 (3H, t), ¹³C NMR (CDCl₃, 100 MHz) δ 170.3, 165.3, 60.7, 58.9, 45.3, 35.5, 28.7, 24.2, 22.5, 15.9, 12.0.
Compound No. 68. white solid, R_{f} 0.26 (1.0:9.0 MeOH/MC), ¹H NMR (DMSO-*d*₆, 400 MHz) δ 7.96 (1H, s), 7.41 (1H, s), 6.90 (1H, s), 4.34 (1H, t, *J* = 6.2 Hz), 4.18 (1H, t, *J* = 7.8 Hz), 3.40-3.35 (1H, m), 3.29-3.27 (1H, m), 2.70 (1H, dd, *J* = 5.6, 16 Hz), 2.28 (1H, dd, *J* = 6.4, 16 Hz), 2.15-2.08 (1H, m), 1.91-1.74 (3H, m), ¹³C NMR (DMSO-*d*₆, 100 MHz) δ 172.1, 170.4, 166.2, 59.2, 52.1, 45.5, 35.3, 28.2, 23.0.
Compound No. 70. white solid, ¹H NMR (CDCl₃, 400 MHz) δ 7.33 (1H, s), 4.20 (1H, t, *J* = 5.6 Hz), 4.10 (1H, t, *J* = 8.2 Hz), 3.61-3.48 (2H, m), 2.69 (2H, t, *J* = 7.0 Hz), 2.41-2.28 (2H, m), 2.10 (3H, s), 2.07-1.93 (3H, m), 1.91-1.84 (1H, m).
Compound No. 80. white solid, ¹H NMR (DMSO-*d₆*, 400 MHz) δ 9.29 (1H, s), 8.10 (1H, d, *J* = 3.2 Hz), 6.89 (2H, d, *J* = 8.4 Hz), 6.65 (2H, d, *J* = 8.0 Hz), 3.90 (1H, d, *J* = 4.4 Hz), 3.42-3.58 (1H, m), 3.20-3.14 (1H, m), 2.92-2.82 (2H, m), 2.76 (1H, dd, *J* = 13.6, 4.8 Hz), 1.98-1.91 (2H, m), 1.80-1.72 (1H, m), 1.61-1.52 (1H, m), ¹³C NMR (DMSO-*d₆*, 100 MHz) δ 168.3, 164.8, 156.3, 130.7, 125.8, 115.0, 58.1, 57.1, 44.5, 38.6, 28.5, 21.2.

### [Examples]

### Example 1. Expression of pathogen resistance-related genes PR-1 and PDF1.2 in thale cress

After treating thale cress with the 2,5-diketopiperazine compound of the present invention, expression of antifungal resistance-inducing genes was measured as described below.

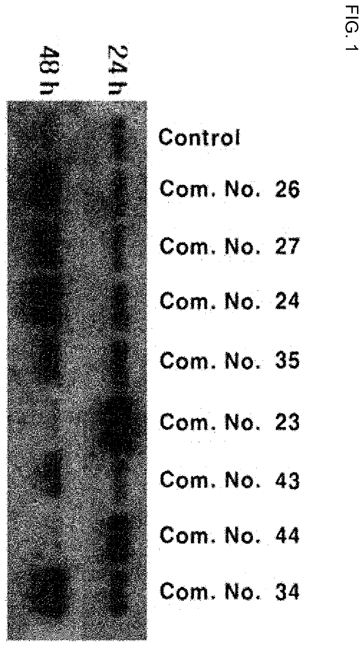

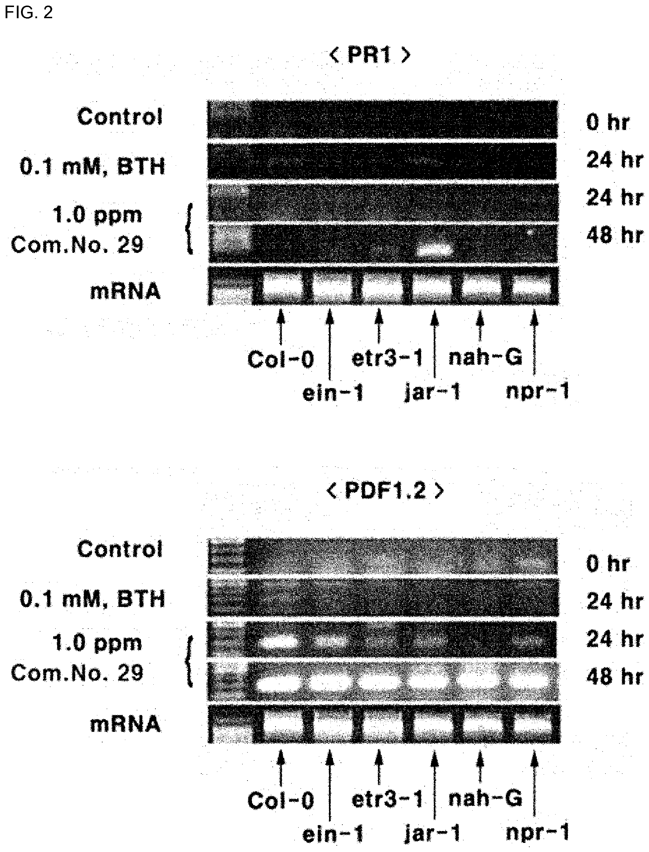

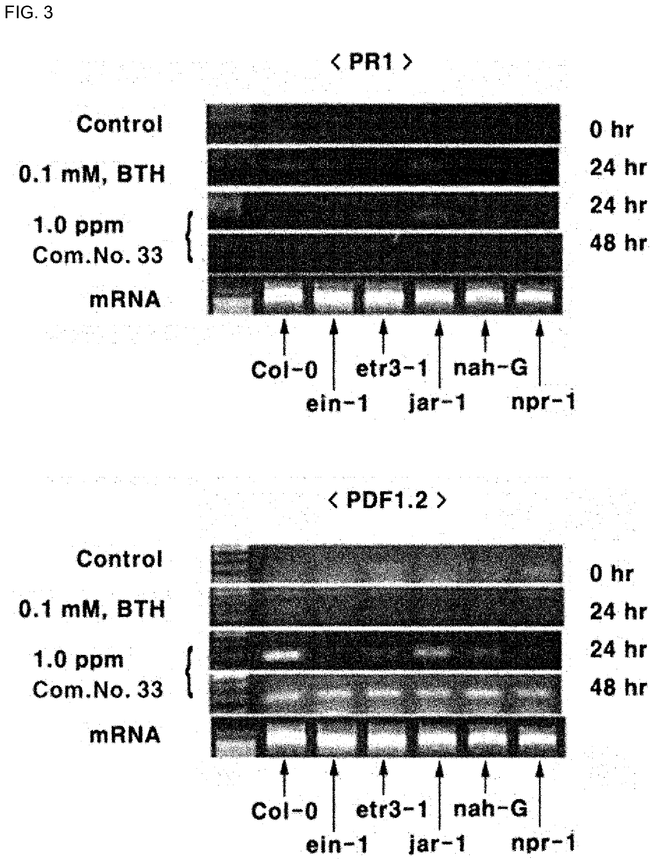

Wild type (Col-0) and transformed (with nahG gene to suppress expression of PR gene) thale cress were used for experiment. Thale cress was obtained from the Ohio State University Stock Center (Ohio State University, Columbus, Ohio, USA). It is known that an NahG-transformed plant encodes salicylate dehydrogenase and degrades salicylic acid (SA). The seed of thale cress was surface-sterilized (immersed in 70% ethanol for 2 minutes and then in 1% sodium hypochlorite for 20 minutes). After washing 4 times with sterilized distilled water, the seed was placed on a Petri dish holding Murashige-Skoog (MS) medium (Gibco BRL) containing 0.8% agar and 1.5% sucrose (pH 5.7). The seed was allowed to sprout at 4 °C for 2 days in the shade. The resulting seedling was kept in a growth cabinet set to a 12 hr/12 hr light/dark cycle under a 40-W fluorescent lamp. Relative humidity and temperature were maintained at 50-60% and 22±1 °C, respectively. 2 weeks later, the seedling was transplanted into a 60-mL pot holding potting soil sterilized twice with steam for 1 hour with a 24-hour interval. The plant was further cultivated in growth chamber maintained at 70% relative humidity with a 9 hr/15 hr light (200 µE/m²s, 24 °C)/dark (20 °C) cycle. The plant was watered every other day and treated with a modified half-strength Hoagland nutrient solution once a week. 2 weeks later, 0.1, 1.0 or 10 ppm of a test compound was applied to the thale cress via soil drench. 12 hours or 24 hours later after the application of test compound to the thale cress via soil drench, leaf tissue was acquired from the plant for RNA analysis. Total RNA of the thale cress was extracted by homogenizing at least 2 g of frozen tissue with an extraction buffer (0.35 M glycine, 0.048 M NaOH, 0.34 M NaCl, 0.04 M EDTA, 4% (w/v) SDS) of the same volume. Subsequently, after extracting the resulting homogeneous suspension with phenol and chloroform, RNA was precipitated with LiCl. RT-PCR was carried out using Ex Taq polymerase (Takara Biomedicals, Japan) according to Kishimoto's method [Kishimoto, K. et al., 2005. Plant Cell Physiol, 46:1093-1102]. A reaction mixture was prepared adding 0.1 µg of cDNA, 10 pmol of forward and reverse primers respectively, 250 nmol of dNTP and 0.5 U of Ex Taq polymerase to 20 µL of a buffer. The PCR was performed with PTC-100 (MJ Research, USA). After denaturation at 94°C for 5 minutes, 25 cycles of 1 minute at 94 °C and 1 minute at 57 °C were conducted. Final extension was performed at 72 °C for 10 minutes. For the PDF1.2 gene, 5'-TGCGGTAACACCGAACCATAC-3' (SEQ ID NO 1) was used as a forward primer and 5'-CGACAGTTGCATTGGTCCTCT-3' (SEQ ID NO 2) was used as a reverse primer. And for the PR-1a gene, 5'-AACCGCCAAAAGCAAACGCA-3' (SEQ ID NO 3) was used as a forward primer and 5'-TCACGGAGGCACAACCAAGTC-3' (SEQ ID NO 4) was used as a reverse primer. The amplified PCR product was analyzed on 1.2% agarose gel (LAS-3000, Fuji Photo Film Co. Ltd., Japan). The result is shown in FIGS. 1-3.

As seen from FIGS. 1-3, in the wild-type thale cress, the test groups treated with the compounds of the present invention showed increased expression of the antifungal genes PR1 and PDF1.2 as compared to the control group. Meanwhile, in the nahG-transformed thale cress incapable of expressing the PR gene, the test groups treated with the compounds of the present invention showed no expression of the PR1 gene but increased expression of the PDF1.2 gene as compared to the control group. The gene expression increased the most in the test group which was treated with the compound of the present invention at a concentration of 1.0 ppm.

### Example 2. PR-1α-GUS activity in tobacco leaf

100 µL of a diluted solution of each test compound was injected into the second leaf of a tobacco seedling with PR-1αpromoter fused to the GUS gene (Xanthi nc) cultivated for 3 weeks using a syringe. 3 days later, the nearby leaf was sampled with a cork borer with 5 mm inner diameter. The sample was put in a 1.5-mL Eppendorf tube, ground after adding 20 µL of GUS extraction buffer and centrifuged at 8000 g for 3 minutes. After adding the same volume of a 2 mM 4-methylumbellifryl-β-glucuronide (MUG) solution to the supernatant, reaction was performed at 37 °C for 1 hour. After adding 960 µL of stop buffer (0.2M sodium carbonate) to make 1 mL, fluorescence was measured using a TKO 100 fluorometer (Hoefer Scientific Instruments, USA). 4-Methylumbelliferone (MU) was used as standard reagent for correction of fluorescence intensity and GUS activity was measured as MU-mM/10 mg sample/hour. The result is shown in Table 2.

**Table 2**

| Effect on number of anthractic lesions and PR-1α-GUS activity in tobacco leaf | | | |
|---|---|---|---|
| Test compounds | | PR-1α-GUS activity (nM MU/10 mg/h) | Number of anthractic lesions |
| Control | | 54 | 240.3 |
| BTH, 1 ppm | | 21,015 | 58.3 |
| Compound No. 26 | 1 ppm | 27,497 | 63.3 |
| | 0.1 ppm | 34,967 | 67.1 |
| LSD (p = 0.05) | | 33,267 | 48.3 |

### Example 3. Effect of inhibiting plant diseases

After treating a plant with the 2,5-diketopiperazine compound of the present invention, the effect of inhibiting plant diseases was evaluated as described below.

12 seeds of tobacco or cucumber were planted in a plastic pot (diameter 10 cm x height 13 cm) and put in a yellow box. 100 µL of a test compound was injected into the first sprouting seed leaf of the cucumber or tobacco. The test compound was treated after dissolving in 20% of methanol to prepare a stock solution (5000) and diluting the stock solution to concentrations of 1,10 and 100 ppm.

7 days later, soft rot-causing *Pectobacterium carotovorum* SCC1 cultured in TSA medium for 27 hours was sprayed onto the leaf of the plant at a concentration of 10⁸ cfu/mL. After cultivating the plant at 30 °C for 3 days, anthracnose-causing *Colletotrichum orbiculare* that had been cultured in GBA (green bean agar) medium for about 2-3 weeks to induce sporing was sprayed at a concentration of 10⁵ cell/mL and the plant was cultivated at 26 °C for 1 day. When the 4th or 5th leaf sprouted, 200 µL of each of the test compound was injected into the 3rd leaf. 7 days later, after spraying the soft rot-causing bacteria onto the leaf of the plant at a concentration of 10⁸ cfu/mL, the plant was cultivated at 30 °C for 3 days.

After visual inspection, the area of soft rot lesion was evaluated from 0 to 100% and the number of anthractic lesions was counted.

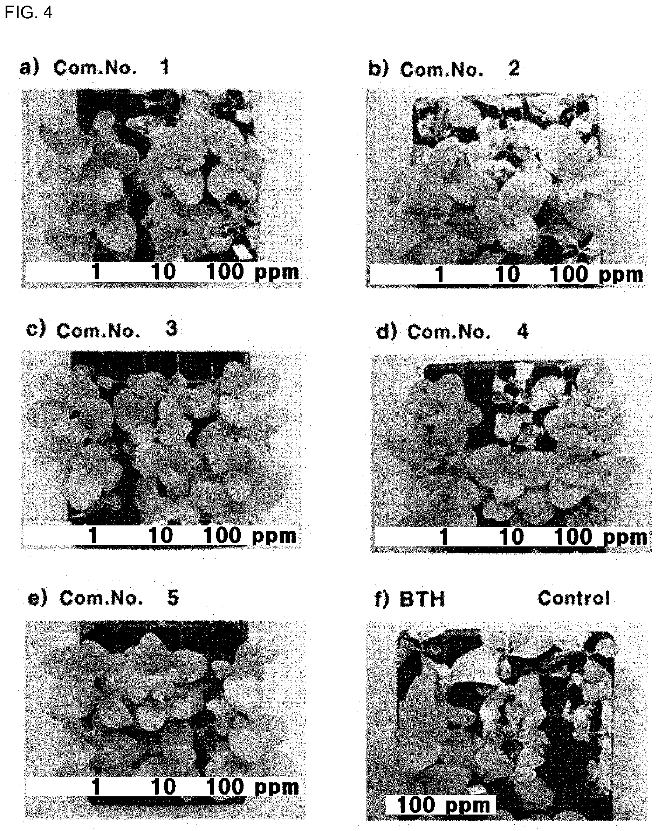

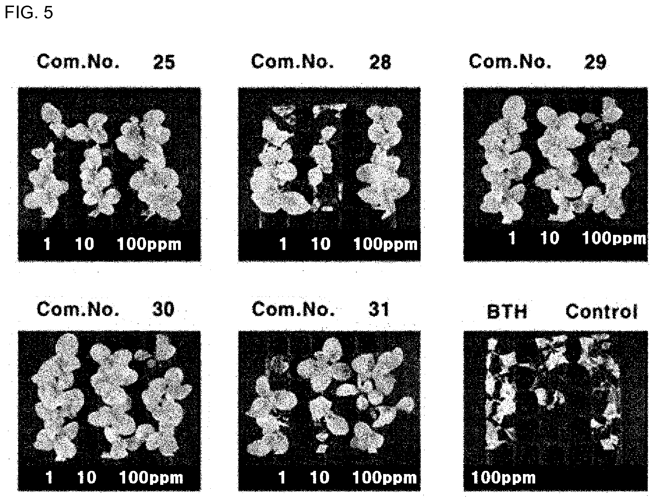

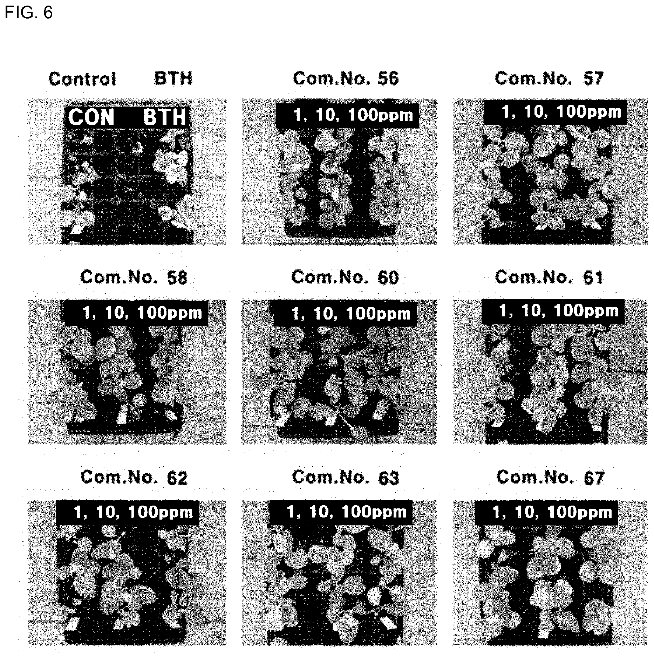

The test result for the effect on the inhibition of soft rot in tobacco is given in Tables 3-5 and FIGS. 4-6.

**Table 3**

| Inhibitory effect against lesion by soft rot-causing *Pectobacterium carotovorum* SCC1 in tobacco leaf | | | |
|---|---|---|---|
| Test compounds | Area of lesion (%) at different concentrations | | |
| | 1 ppm | 10 ppm | 100 ppm |
| Compound No. 1 | 10 | 32.5 | 50 |
| Compound No. 2 | 32.5 | 40 | 60 |
| Compound No. 3 | 20 | 10 | 0 |
| Compound No. 4 | 17.5 | 52.5 | 52.5 |
| Compound No. 5 | 2.5 | 5 | 40 |
| BTH | 52.5 | 52.5 | 52.5 |
| Control | 87.5 | 87.5 | 87.5 |
| LSD (p = 0.05) | 23.5 | 18.2 | 18.8 |

**Table 4**

| Inhibitory effect against lesion by soft rot-causing *Pectobacterium carotovorum* SCC1 in tobacco leaf | | | |
|---|---|---|---|
| Test compounds | Area of lesion (%) at different concentrations | | |
| | 1 ppm | 10 ppm | 100 ppm |
| Compound No. 25 | 15 | 12.5 | 0 |
| Compound No. 28 | 12.5 | 82.5 | 2.5 |
| Compound No. 29 | 27.5 | 0 | 0 |
| Compound No. 30 | 2.5 | 7.5 | 2.5 |
| Compound No. 31 | 47.5 | 60 | 17.5 |
| BTH | 95 | 95 | 95 |
| Control | 100 | 100 | 100 |
| LSD (p = 0.05) | 39.9 | 36.2 | 17.4 |

**Table 5**

| Inhibitory effect against lesion by soft rot-causing *Pectobacterium carotovorum* SCC1 in tobacco leaf | | | |
|---|---|---|---|
| Test compounds | Area of lesion (%) at different concentrations | | |
| | 1 ppm | 10 ppm | 100 ppm |
| Compound No. 56 | 7.5 | 10 | 10 |
| Compound No. 57 | 10 | 10 | 12.5 |
| Compound No. 58 | 35 | 25 | 7.5 |
| Compound No. 59 | 12.5 | 32.5 | 47.5 |
| Compound No. 60 | 33.7 | 25 | 7.5 |
| Compound No. 61 | 10 | 10 | 17.5 |
| Compound No. 62 | 40 | 12.5 | 10 |
| Compound No. 63 | 15 | 22.5 | 65 |
| Compound No. 64 | 55 | 37.5 | 60 |
| Compound No. 65 | 60 | 57.5 | 52.5 |
| Compound No. 66 | 35 | 37.5 | 37.5 |
| Compound No. 67 | 25 | 35 | 40 |
| Compound No. 68 | 10 | 7.5 | 30 |
| Control | 73.75 | 73.75 | 73.75 |
| BTH | 50 | 50 | 50 |
| LSD (p = 0.05) | 43 | 43 | 45 |

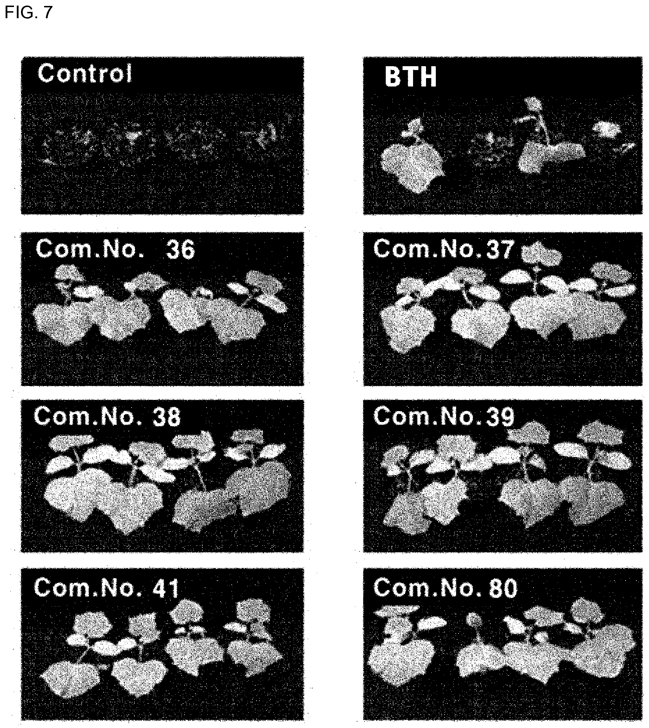

The test result for the effect on the inhibition of soft rot in cucumber is given in Tables 6-8 and FIG. 7.

**Table 6**

| Inhibitory effect against lesion by soft rot-causing *Pectobacterium carotovorum* SCC1 in cucumber leaf | | | |
|---|---|---|---|
| Test compounds | Area of lesion (%) at different concentrations | | |
| | 1 ppm | 10 ppm | 100 ppm |
| Compound No. 1 | 95 | 77.5 | 50 |
| Compound No. 2 | 92.5 | 82.5 | 50 |
| Compound No. 3 | 45 | 45 | 42.5 |
| Compound No. 4 | 37.5 | 45 | 87.5 |
| BTH | 100 | 100 | 100 |
| Control | 100 | 100 | 100 |
| LSD (p = 0.05) | 13.8 | 31.8 | 19.14 |

**Table 7**

| Inhibitory effect against lesion by soft rot-causing *Pectobacterium carotovorum* SCClin cucumber leaf | | | |
|---|---|---|---|
| Test compounds | Area of lesion (%) at different concentrations | | |
| | 1 ppm | 10 ppm | 100 ppm |
| Compound No. 25 | 78 | 73 | 85 |
| Compound No. 28 | 55 | 83 | 65 |
| Compound No. 29 | 43 | 63 | 90 |
| Compound No. 30 | 88 | 75 | 70 |
| Compound No. 31 | 95 | 78 | 80 |
| BTH | 90 | 90 | 90 |
| Control | 100 | 100 | 100 |
| LSD (p = 0.05) | 36 | 33 | 30 |

**Table 8**

| **I**nhibitory effect against lesion by soft rot-causing *Pectobacterium carotovorum* SCC1 in cucumber leaf | | | |
|---|---|---|---|
| Test compounds | Area of lesion (%) at different concentrations | | |
| | 1 ppm | 10 ppm | 100 ppm |
| Compound No. 56 | 5 | 80 | 70 |
| Compound No. 57 | 70 | 72.5 | 60 |
| Compound No. 58 | 75 | 57.5 | 80 |
| Compound No. 59 | 30 | 32.5 | 80 |
| Compound No. 60 | 50 | 60 | 27.5 |
| Compound No. 61 | 5 | 70 | 75 |
| Compound No. 63 | 82.5 | 42.5 | 50 |
| Compound No. 64 | 50 | 55 | 65 |
| BTH | 100 | 100 | 100 |
| Control | 100 | 100 | 100 |
| LSD (p = 0.05) | 42.3 | 53.2 | 39.9 |

The test result for the effect on the inhibition of anthracnose in cucumber is given in Table 9.

**Table 9**

| Inhibitory effect against anthractic lesion by *Colletotrichum orbiculare* in first and second leaves of cucumber | | | | | | | | |
|---|---|---|---|---|---|---|---|---|
| Test compounds | Number of anthractic lesions in first leaf | | | Number of anthractic lesions in second leaf | | | | |
| | 100 ppm | 10 ppm | 1 ppm | 0.1 ppm | 100 ppm | 10 ppm | 1 ppm | 0.1 ppm |
| Compound No. 25 | 2.0 | 1.0 | 1.3 | 3.8 | 27.8 | 22.5 | 46.0 | 37.0 |
| Compound No. 28 | 10.8 | 12.3 | 2.3 | 5.3 | 77.3 | 87.3 | 62.3 | 55.3 |
| Compound No. 29 | 5.0 | 10.0 | 5.0 | 0.8 | 87.0 | 55.5 | 35.8 | 51.3 |
| Compound No. 30 | 7.8 | 7.0 | 3.0 | 2.5 | 55.3 | 35.3 | 39.0 | 66.8 |
| BTH | 0.8 | 0.8 | 0.8 | 0.8 | 100.3 | 100.3 | 100.3 | 100.3 |
| Control | 12.5 | 12.5 | 12.5 | 12.5 | 96.8 | 96.8 | 96.8 | 96.8 |
| LSD (p = 0.05) | 7.7 | 8.7 | 5.3 | 5.4 | 67.3 | 55.1 | 34.7 | 34.0 |

As seen from Tables 3-9, the test groups treated with the 2,5-ketopiperazine compounds exhibited superior resistance to plant diseases caused by the soft rot-bacteria or *Colletotrichum acutatum* in tobacco and cucumber as compared to the control groups. In particular, the test groups treated at concentrations of 10-100 ppm showed very superior disease resistance.

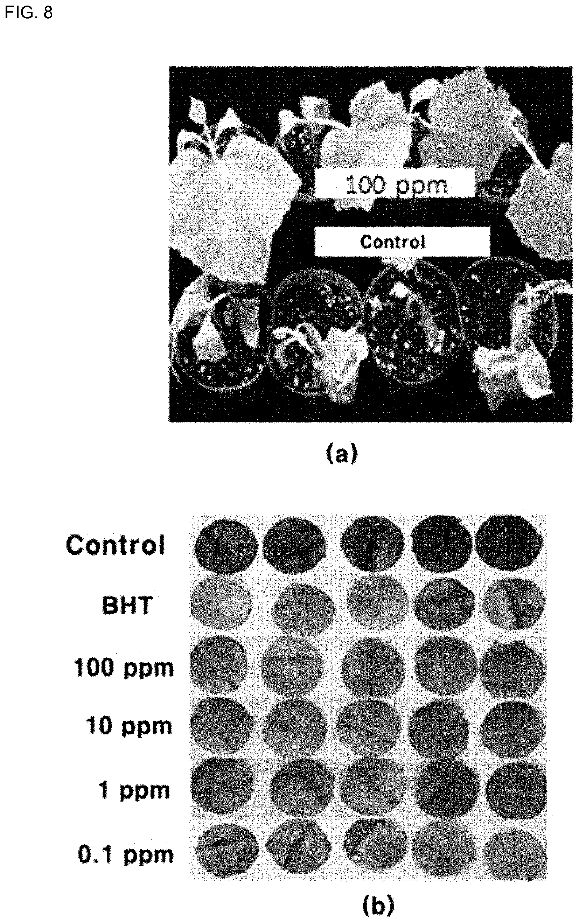

And, as seen from FIGS. 4-8, the test groups treated with the 2,5-ketopiperazine compounds showed excellent resistance to plant diseases as compared to the control groups or those treated with the control agent benzo-1,2,3-thiadiazole-7-carbothioic acid S-methyl ester (BTH).

Table 9 shows the result of testing for the first and second leaves to investigate whether the effect of the agent is maintained for a certain period of time. It was observed that the effect of the agricultural agents of the present invention was relatively highly maintained in the second leaf as compared to the control agent (BHT). Accordingly, it can be seen that the effect of the agricultural agent of the present invention is maintained for a long time.

### Example 5. Effect of inhibiting anthracnose in red pepper fruit

The following experiment was carried out in order to investigate the systemic effect owing to the resistance to plant diseases induced by the agricultural agent of the present invention.

30-cm tall red pepper seedlings (6 weeks) were treated with 0.1, 1.0 or 10.0 ppm of test compounds via soil drench. 10 days later, red pepper fruits were picked and put in humidified plastic boxes. To induce anthracnose, 10 µL of a spore suspension of *Colletotrichum acutatum* (1x10⁵ conidia/mL) was dropped onto the surface of each red pepper fruit. After a week under a humidified condition at 28 °C, appearance of anthracnose was examined.

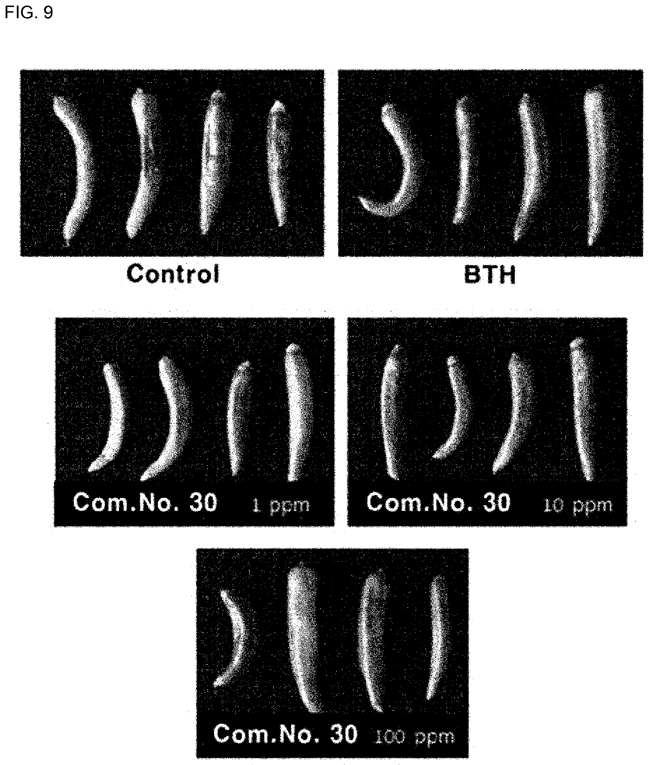

The result of testing the inhibitory effect against anthracnose in red pepper fruit is given in Table 10 and FIG. 9.

**Table 10**

| Inhibitory effect against lesion by anthracnose-causing *Colletotrichum acutatum* in red pepper fruit | | | |
|---|---|---|---|
| Test compounds | Appearance of anthracnose (%) | | |
| | 1 ppm | 10 ppm | 100 ppm |
| Compound No. 25 | 0 | 25 | 25 |
| Compound No. 28 | 0 | 25 | 0 |
| Compound No. 29 | 0 | 0 | 0 |
| Compound No. 30 | 0 | 0 | 0 |
| Compound No. 31 | 25 | 25 | 0 |
| BTH | 25 | 0 | 0 |
| Control | 50 | 50 | 50 |

As seen from Table 10, the test groups treated with the compounds of the present invention showed significantly lower appearance of anthracnose as compared to the control groups. As seen from FIG. 9, anthractic lesion was observed in the control groups but it was hardly observed in the group treated with Compound No. 30. Accordingly, it can be seen that, even when treated on soil, the effect of the agricultural agent of the present invention is observed also in the fruit.

Example 6. Effect of inhibiting soft rot in Chinese cabbage hydrocultured in summer

The following experiment was carried out in order to investigate whether the compound of the present invention is effective also for bacterial soft rot.

After spraying test compounds to hydrocultured Chinese cabbage, naturally occurring soft rot was examined 1 week later. Chinese cabbage is a low-temperature vegetable and shows very poor growth as well as severe bacterial soft rot caused by *Pectobacterium carotovorum SCC1* in hot summer season. Thus, without artificially inducing soft rot, area of lesion in the leaf was investigated for 3-4 leaves to ensure statistical uniformity.

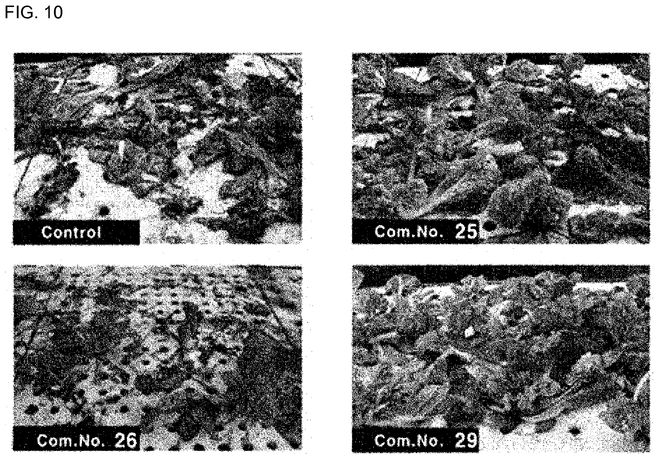

The result of testing the inhibitory effect against bacterial soft rot in Chinese cabbage leaf is given in Table 11 and FIG. 10.

**Table 11**

| Inhibitory effect against soft rot by *Pectobacterium carotovorum SCC1* in Chinese cabbage hydrocultured in summer | |
|---|---|
| Test compounds | Appearance of soft rot (%) |
| Compound No. 25 (20 ppm) | 19 |
| Compound No. 28 (20 ppm) | 49 |
| Compound No. 29 (20 ppm) | 14 |
| Control | 58 |
| LSD (p = 0.05) | 7 |

As seen from Table 11 and FIG. 10, the compounds of the present invention showed the effect of significantly inhibiting soft rot in Chinese cabbage. In particular, Com. No. 25 and 29 showed very superior inhibitory effect against soft rot in Chinese cabbage. Accordingly, it can be seen that the compound of the present invention is useful also to the plants whose growth environment is very poor.

### [Formulation Examples]

Representative formulation examples of the agricultural agent comprising the compound represented by Chemical Formula 1 as an active ingredient for different purposes are provided in the followings.

### Formulation Example 1. Wettable powder

10 g the compound of Chemical Formula 1, 10 g of NK250L (surfactant), 10 g of white carbon and 70 g of pyrophyllite (extender) were mixed and ground to prepare a wettable powder.

### Formulation Example 2. Emulsifiable concentrate

10 g the compound of Chemical Formula 1, 10 g of DDY2000 (surfactant) and 80 g of xylene were mixed to prepare an emulsifiable concentrate.

### Formulation Example 3. Suspendable concentrate

10 g the compound of Chemical Formula 1, 10 g of HY1910 (surfactant), 5 g of propylene glycol, 0.2 g of xanthan gum, 0.15 g of KM-73 (antifoaming agent), 0.2 g of biocide LS (antiseptic), 0.1 g of KNP (thickener) and 74.35 g of water (extender) were mixed and ground in a ball mill to prepare a suspendable concentrate.

### Formulation Example 4. Floating granule

5 g of the compound of Chemical Formula 1, 7.5 g of paraffin oil, 2 g of sodium alkylsulfosuccinate (surfactant), 3 g of white carbon, 1.2 g of xanthan gum, 0.8 g of sodium polyacrylate and 80.5 g of potassium chloride were mixed, granulated with a reciprocating extruder and then dried to prepare a floating granule.

### Formulation Example 5. Granule

5 g of the compound of Chemical Formula 1, 2.5 g of HY1910 (surfactant), 0.2 g of NK250L (surfactant), 0.5 g of soda ash, 2.0 g of dextrin, 25 g of bentonite and 64.8 g of talc were kneaded with water, granulated with a reciprocating extruder and then dried to prepare a granule.

As described in detail above, the agricultural agent of the present invention exhibits excellent effect of controlling plant diseases caused by bacteria, viruses or fungi such as soft rot, damping off, blight, withering, spot or mosaic disease.

The present invention has been described in detail with reference to specific embodiments thereof.

## Claims

1. Use of an agricultural agent for providing induced systemic resistance (ISR) against plant diseases, which comprises a 2,5-diketopiperazine compound selected from the following or an agriculturally acceptable salt thereof as an active ingredient:
3-hydroxymethyl-6-(2-methylpropyl)-2,5-diketopiperazine,
3-(2-methylpropyl)-2,5-diketopiperazine,
3-(1-hydroxyethyl)-6-(2-methylpropyl)-2,5-diketopiperazine,
3-isopropyl-2,5-diketopiperazine,
3-(indol-3-ylmethyl)-2,5-diketopiperazine,
2,5-diketopiperazine,
3-methyl-2,5-diketopiperazine,
3,6-dimethyl-2,5-diketopiperazine,
3-methyl-6-(2-methylethyl)-2,5-diketopiperazine,
3-methyl-6-(4-hydroxybenzyl)-2,5-diketopiperazine,
3-(1-hydroxyethyl)-6-(2-mercaptomethyl)-2,5-diketopiperazine,
3-(1-hydroxyethyl)-6-(2-methylthiomethyl)-2,5-diketopiperazine,
3-(1-hydroxyethyl)-6-(2-tritylthiomethyl)-2,5-diketopiperazine,
3-(1-hydroxyethyl)-6-benzyl-2,5-diketopiperazine,
3-(7-hydroxyethyl)-6-(4-hydroxybenzyl)-2,5-diketopiperazine,
3-(1-hydroxyethyl)-6-(indol-3-ylmethyl)-2,5-diketopiperazine,
3-(2-methylpropyl)-6-(2-mercaptornethyl)-2,5-diketopiperazine,
3-(2-methylpropyl)-6-(2-methylthiomethyl)-2,5-diketopiperazine,
3-(2-methylpropyl)-6-(2-tritylthiamethyl)-2,5-diketopiperazine,
3-(2-methylpropyl)-6-benzyl-2,5-diketopiperazine,
3-(2-methylpropyl)-6-(4-hydroxybenzyl)-2,5-diketopiperazine,
3-(2-methylpropyl)-6-(indol-3-ylmethyl)-2,5-diketopiperazine,
3-(4-hydroxybenzyl)hexahydropyrrolo[1,2-a]pyrazin-1,4-dione,
3-benzylhexahydropyrrolo[1,2-a]pyrazin-1,4-dione,
3-methylhexahydropyrrolo[1,2-a]pyrazin-1,4-dione,
3-isopropylhexahydropyrrolo[1,2-a]pyrazin-1,4-dione,
3-(2-methylpropyl)hexahydropyrrolo[1,2-a]pyrazin-1,4-dione,
3-(1-methylpropyl)hexahydropyrrolo[1,2-a]pyrazin-1,4-dione, hexahydropyrrolo[1,2-a]pyrazin-1,4-dione,
3-(indol-3-ylmethyl)hexahydropyrrolo[2,2-a]pyrazin-1,4-dione, octahydropyrrolo[1,2-a:1',2'-d]pyrazin-5,10-dione,
3-hydroxymethylhexahydropyrrolo[1,2-a]pyrazin-1,4-dione,
3-(1-hydroxyethyl)hexahydropyrrolo[1,2-a]pyrazin-1,4-dione,
3-(2-carbamoylethyl)hexahydropyrrolo[1/2-a]pyrazin-1/4-dione,
3-(2-methylthioethyl)hexahydropyrrolo[1,2-a]pyrazin-1,4-dione,
3-carbamoylmethylhexahydropyrrolo[1,2-a]pyrazin-1,4-dione,
3-mercaptomethylhexahydropyrrolo[1,2-a]pyrazin-1,4-dione,
3-methylthiomethylhexahydropyrrolo[1,2-a] pyrazin-1,4-dione,
3-tritylthiomethylhexahydropyrrolo[1,2-a] pyrazin-1,4-dione,
3-(2-methoxycarbonylethyl)hexahydropyrrolo[1,2-a]pyrazin-1,4-dione,
3-(2-methoxycarbonylmethyl)hexahydropyrrolo[1,2-a]pyrazin-1,4-dione,
3-(2-aminoethyl)hexahydropyrrolo[1,2-a]pyrazin-1,4-dione,
3-(2-carboxyethyl)hexahydropyrrolo[1,2-a]pyrazin-1,4-dione,
3-(2-carboxymethyl)hexahydropyrrolo[1,2-a]pyrazin-1,4-dione,
3-(imidazol-4-ylmethyl)hexahydropyrrolo[1,2-a]pyrazin-1,4-dione and
3-(2-acetylaminoethyl)hexahydropyrrolo[1,2-a]pyrazin-1,4-dione.

2. Use according to claim 1, wherein the agricultural agent comprises 0.001 to 99 wt.-% of the active ingredient and an excipient as balance.

3. Use according to one of claims 1 or 2, wherein the agricultural agent is formulated into a wettable powder, a suspension, an emulsifiable concentrate, an emulsion, a mucroemulsion, a soluble concentrate, a dispersible concentrate, a watcr-dispersible granule, a granule, a dustable powder, a suspendable concentrate, a water-dispersible granule, a floating granule or a tablet.

4. Use according to one of claims 1 to 3, wherein the plant disease is soft rot, damping off, blight, withering, spot or mosaic disease.

5. Use according to one of claims 1 to 4, wherein the plant is a dicotyledon.

6. Use according to claim 5, wherein the dicotyledon is selected from tobacco, Chinese cabbage, red pepper, cucumber, potato and tomato.

## Patentansprüche

1. Verwendung eines landwirtschaftlichen Mittels zum Verleihen von induzierter systemischer Widerstandsfähigkeit ("Induced Systemic Resistance (ISR)") gegen Pflanzenkrankheiten, welches eine 2,5-Diketopiperazinverbindung, welche aus den folgenden ausgewählt ist, oder ein landwirtschaftlich verträgliches Salz davon als wirksamen Bestandteil enthält:
3-Hydroxymethyl-6-(2-methylpropyl)-2,5-diketopiperazin,
3-(2-Methylpropyl)-2,5-diketopiperazin,
3-(1-Hydroxyethyl)-6-(2-methylpropyl)-2,5-diketopiperazin,
3-Isopropyl-2,5-diketopiperazin,
3-(Indol-3-ylmethyl)-2,5-diketopiperazin,
2,5-Diketopiperazin,
3-Methyl-2,5-diketopiperazin,
3,6-Dimethyl-2,5-diketopiperazin,
3-Methyl-6-(2-methylethyl)-2,5-diketopiperazin,
3-Methyl-6-(4-hydroxybenzyl)-2,5-diketopiperazin,
3-(1-Hydroxyethyl)-6-(2-mercaptomethyl)-2,5-diketopiperazin,
3-(1-Hydroxyethyl)-6-(2-methylthiomethyl)-2,5-diketopiperazin,
3-(1-Hydroxyethyl)-6-(2-tritylthiomethyl)-2,5-diketopiperazin,
3-(1-Hydroxyethyl)-6-benzyl-2,5-diketopiperazin,
3-(1-Hydroxyethyl)-6-(4-hydroxybenzyl)-2,5-diketopiperazin,
3-(1-Hydroxyethyl)-6-(indol-3-ylmethyl)-2,5-diketopiperazin,
3-(2-Methylpropyl)-6-(2-mercaptomethyl)-2,5-diketopiperazin,
3-(2-Methylpropyl)-6-(2-methylthiomethyl)-2,5-diketopiperazin,
3-(2-Methylpropyl)-6-(2-tritylthiomethyl)-2,5-diketopiperazin,
3-(2-Methylpropyl)-6-benzyl-2,5-diketopiperazin,
3-(2-Methylpropyl)-6-(4-hydroxybenzyl)-2,5-diketopiperazin,
3-(2-Methylpropyl)-6-(indol-3-ylmethyl)-2,5-diketopiperazin,
3-(4-Hydroxybenzyl)hexahydropyrrol[1,2-a]pyrazin-1,4-dion,
3-Benzylhexahydropyrrol[1,2-a]pyrazin-1,4-dion,
3-Methylhexahydropyrrol[1,2-a]pyrazin-1,4-dion,
3-Isopropylhexahydropyrrol[1,2-a]pyrazin-1,4-dion,
3-(2-Methylpropyl)hexahydropyrrol[1,2-a]pyrazin-1,4-dion,
3-(1-Methylpropyl)hexahydropyrrol[1,2-a]pyrazin-1,4-dion, Hexahydropyrrol[1,2-a]pyrazin-1,4-dion,
3-(Indol-3-ylmethyl)hexahydropyrrol[1,2-a]pyrazin-1,4-dion, Octahydropyrrol[1,2-a:1',2'-d]pyrazin-5,10-dion,
3-Hydroxymethylhexahydropyrrol[1,2-a]pyrazin-1,4-dion,
3-(1-Hydroxyethyl)hexahydropyrrol[1,2-a]pyrazin-1,4-dion,
3-(2-Carbamoylethyl)hexahydropyrrol[1,2-a]pyrazin-1,4-dion,
3-(2-Methylthioethyl)hexahydropyrrol[1,2-a]pyrazin-1,4-dion,
3-Carbamoylmethylhexahydropyrrol[1,2-a]pyrazin-1,4-dion,
3-Mercaptomethylhexahydropyrrol[1,2-a]pyrazin-1,4-dion,
3-Methylthiomethylhexahydropyrrol[1,2-a]pyrazin-1,4-dion,
3-Tritylthiomethylhexahydropyrrol[1,2-a]pyrazin-1,4-dion,
3-(2-Methoxycarbonylethyl)hexahydropyrrol[1,2-a]pyrazin-1,4-dion,
3-(2-Methoxycarbonylmethyl)hexahydropyrrol[1,2-a]pyrazin-1,4-dion,
3-(2-Aminoethyl)hexahydropyrrol[1,2-a]pyrazin-1,4-dion,
3-(2-Carboxyethyl)hexahydropyrrol[1,2-a]pyrazin-1,4-dion,
3-(2-Carboxymethyl)hexahydropyrrol[1,2-a]pyrazin-1,4-dion,
3-(Imidazol-4-ylmethyl)hexahydropyrrol[1,2-a]pyrazin-1,4-dion und
3-(2-Acetylaminoethyl)hexahydropyrrol[1,2-a]pyrazin-1,4-dion.

2. Verwendung nach Anspruch 1, wobei das landwirtschaftliche Mittel 0,001 bis 99 Gew.-% des wirkenden Bestandteils und einen Hilfsstoff als Rest aufweist.

3. Verwendung nach einem der Ansprüche 1 oder 2, wobei das landwirtschaftliche Mittel in ein benetzbares Pulver, eine Suspension, ein emulgierbares Konzentrat, eine Emulsion, eine Mikroemulsion, ein lösbares Konzentrat, ein dispergierbares Konzentrat, ein wasserdispergierbares Granulat, ein Granulat, ein zerstäubbares Pulver, ein suspendierbares Konzentrat, ein wasserdispergierbares Granulat, ein fließfähiges Granulat oder eine Tablette formuliert ist.

4. Verwendung nach einem der Ansprüche 1 bis 3, wobei die Pflanzenkrankheit Nassfäule, Keimlingsfäule, Weißfäule bzw. Braunfäule, Trockenfäule, Flecken- oder Mosaikkrankheit ist.

5. Verwendung nach einem der Ansprüche 1 bis 4, wobei die Pflanze eine zweikeimblättrige Pflanze ist.

6. Verwendung nach Anspruch 5, wobei die zweikeimblättrige Pflanze ausgewählt ist aus Tabak, Chinakohl, roter Paprika bzw. rote Peperoni, Gurke, Kartoffel und Tomate.

## Revendications

1. Utilisation d'un agent agricole pour fournir une résistance systémique induite contre des maladies végétales, qui comprend un composé 2,5-dicétopipérazine sélectionné parmi les suivants, ou un sel de celui-ci acceptable sur le plan agricole en tant qu'ingrédient actif :
3-hydroxyméthyl-6-(2-méthylpropyl)-2,5,dicétopipérazine,
3-(2-méthylpropyl)-2,5-dicétopipérazine,
3-(1-hydroxyéthyl)-6-(2-méthylpropyl)-2,5-dicétopipérazine,
3-isopropyl-2,5-dicétopipérazine,
3-(indol-3-ylméthyl)-2,5-dicétopipérazine,
2,5-dicétopipérazine,
3-méthyl-2,5-dicétopipérazine,
3,6-diméthyl-2,5-dicétopipérazine,
3-méthyl-6-(2-méthyléthyl)-2,5-dicétopipérazine,
3-méthyl-6-(4-hydroxybenzyl)-2,5-dicétopipérazine,
3-(1-hydroxyéthyl)-6-(2-mercaptométhyl)-2,5-dicétopipérazine,
3-(1-hydroxyéthyl)-6-(2-méthylthiométhyl)-2,5-dicétopipérazine,
3-(1-hydroxyéthyl)-6-(2-tritylthiométhyl)-2,5-dicétopipérazine,
3-(1-hydroxyéthyl)-6-benzyl-2,5-dicétopipérazine,
3-(1-hydroxyéthyl)-6-(4-hydroxybenzyl)-2,5-dicétopipérazine,
3-(1-hydroxyéthyl)-6-(indol-3-ylméthyl)-2,5-dicétopipérazine,
3-(2-méthylpropyl)-6-(2-mercaptométhyl)-2,5-dicétopipérazine,
3-(2-méthylpropyl)-6-(2-méthylthiométhyl)-2,5-dicétopipérazine,
3-(2-méthylpropyl)-6-(2-tritylthiométhyl)-2,5-dicétopipérazine,
3-(2-méthylpropyl)-6-benzyl-2,5-dicétopipérazine,
3-(2-méthylpropyl)-6-(4-hydroxybenzyl)-2,5-dicétopipérazine,
3-(2-méthylpropyl)-6-(indol-3-ylméthyl)-2,5-dicétopipérazine,
3-(4-hydroxybenzyl)hexahydropyrrolo[1,2-a]pyrazin-1,4-dione,
3-benzylhexahydropyrrolo[1,2-a]pyrazin-1,4-dione,
3-méthylhexahydropyrrolo[1,2-a]pyrazin-1,4-dione,
3-isopropylhexahydropyrrolo[1,2-a]pyrazin-1,4-dione,
3-(2-méthylpropyl)hexahydropyrrolo[1,2-a]pyrazin-1,4-dione,
3-(1-méthylpropyl)hexahydropyrrolo[1,2-a]pyrazin-1,4-dione, hexahydropyrrolo[1,2-a]pyrazin-1,4-dione,
3-(indol-3-ylméthyl)hexahydropyrrolo[1,2-a]pyrazin-1,4-dione, octahydropyrrolo[1,2-a: 1',2'-d]pyrazin-5,10-dione,
3-hydroxyméthylhexahydropyrrolo[1,2-a]pyrazin-1,4-dione,
3-(1-hydroxyéthyl)hexahydropyrrolo[1,2-a]pyrazin-1,4-dione,
3-(2-carbamoyléthyl)hexahydropyrrolo[1,2-a]pyrazin-1,4-dione,
3-(2-méthylthioéthyl)hexahydropyrrolo[1,2-a]pyrazin-1,4-dione,
3-carbamoylméthylhexahydropyrrolo[1,2-a]pyrazin-1,4-dione,
3-mercaptométhylhexahydropyrrolo[1,2-a]pyrazin-1,4-dione,
3-méthylthiométhylhexahydropyrrolo[1,2-a]pyrazin-1,4-dione,
3-tritylthiométhylhexahydropyrrolo[1,2-a]pyrazin-1,4-dione,
3-(2-méthoxycarbonyléthyl)hexahydropyrrolo[1,2-a]pyrazin-1,4-dione,
3-(2-méthoxycarbonylméthyl)hexahydropyrrolo[1,2-a]pyrazin-1,4-dione,
3-(2-aminoéthyl)hexahydropyrrolo[1,2-a]pyrazin-1,4-dione,
3-(2-carboxyéthyl)hexahydropyrrolo[1,2-a]pyrazin-1,4-dione,
3-(2-carboxyméthyl)hexahydropyrrolo[1,2-a]pyrazin-1,4-dione,
3-(imidazol-4-ylméthyl)hexahydropyrrolo[1,2-a]pyrazin-1,4-dione, et
3-(2-acétylaminoéthyl)hexahydropyrrolo[1,2-a]pyrazin-1,4-dione.

2. Utilisation selon la revendication 1, dans laquelle l'agent agricole comprend 0,001 à 99 % en poids de l'ingrédient actif et un excipient pour le reste.

3. Utilisation selon l'une des revendications 1 ou 2, dans laquelle l'agent agricole est formulé en une poudre mouillable, une suspension, un concentré émulsionnable, une émulsion, une microémulsion, un concentré soluble, un concentré dispersible, un granulé dispersible dans l'eau, un granulé, une poudre à poudrer, un concentré pouvant être mis en suspension, un granulé flottant ou un comprimé.

4. Utilisation selon l'une des revendications 1 à 3, dans laquelle la maladie végétale est la marciaume molle, la fonte des semis, la rouille, la flétrissure ou la mosaïque.

5. Utilisation selon l'une des revendications 1 à 4, dans laquelle la plante est une dicotylédone.

6. Utilisation selon la revendication 5, dans laquelle la dicotylédone est sélectionnée parmi le tabac, le chou chinois, le piment rouge, le concombre, la pomme de terre et la tomate.
